# EUROPÄISCHE PATENTSCHRIFT

(11) **EP 4 552 684 B1**
(45) Veröffentlichungstag und Bekanntmachung des Hinweises auf die Patenterteilung: **11.11.2026**
(21) Anmeldenummer: 25156130.4
(22) Anmeldetag: 17.09.2024
(51) Int. Cl.: A61N 1/04, A61N 1/36

(54) **VORRICHTUNG UND SYSTEM ZUR ELEKTROSTIMULATION DES AUGES**
APPARATUS AND SYSTEM FOR ELECTROSTIMULATION OF THE EYE
DISPOSITIF ET SYSTÈME D'ÉLECTROSTIMULATION DE L'OEIL

(30) Priorität: 18.09.2023 DE 102023125136
(43) Veröffentlichungstag der Anmeldung: 14.05.2025
(62) Dokumentnummer(n) der früheren Anmeldung(en) nach Art. 76 EPÜ: 24773373.6 / 4 547 315
(73) Patentinhaber: Okuvision GmbH, 72770 Reutlingen (DE)
(72) Erfinder: Berenbold, Steffen, 79289 Horben (DE); Ceretto Gianone, Nicolò, 10129 Torino (TO) (IT); Henzler, Paul, 72622 Nürtingen (DE); Stölzel, Dorothee, 72108 Rottenburg (DE); Kaiser, Fabian, 72790 Pfullingen (DE)
(74) Vertreter: Witte, Weller & Partner Patentanwälte mbB

(56) Entgegenhaltungen:
- WO-A1-2015/104313
- US-A1- 2018 318 586
- US-A1- 2022 305 265
- US-A1- 2023 082 686

## Beschreibung

Die vorliegende Erfindung betrifft eine Vorrichtung, insbesondere zur Elektrostimulation des Auges, und ein System zur Elektrostimulation des Auges, insbesondere zur transkornealen Elektrostimulation, wobei die Vorrichtung ein brillenartiges Gestell mit einem Nasenteil, eine mit dem Nasenteil verbundene Anordnung zum Halten des Gestells an dem Kopf des Nutzers sowie zumindest einen an dem Gestellt angeordneten Halter für ein in Eingriff mit einem Auge des Nutzers zu bringendes Element, insbesondere für eine drahtförmige Stimulationselektrode aufweist.

Aus der WO 2011/086150 A2 sind Stimulationselektroden, Elektrodenhalter sowie Vorrichtungen zur Elektrostimulation grundsätzlich bekannt.

Die bekannte Stimulationselektrode ist eine drahtförmige Elektrode, die an dem brillenartigen Gestell befestigt wird. Wenn das Gestell an dem Kopf eines Patienten angeordnet wird, so gelangt die nachgiebige Drahtelektrode beim Aufsetzen des brillenartigen Gestells in Kontakt mit der vorderen Augenoberfläche im unteren Randbereich der Cornea des Auges. Eine Gegenelektrode ist entweder an dem Gestell vorgesehen oder wird gesondert am Patienten angebracht, beispielsweise an der Stirn des Kopfes oder an einem Ohrläppchen.

Über die Drahtelektrode und die Gegenelektrode kann ein pulsartiges elektrisches Stimulationssignal in das Auge geleitet werden, was dazu führt, dass ein Stimulationsstrom über die Cornea in das Auge fließt.

Die bekannte sowie andere Vorrichtungen werden eingesetzt, um elektrische Stimulationssignale in das Auge zu leiten, weil sich herausgestellt hat, dass dadurch bestimmte Formen der Retinopathia pigmentosa und andere Augenkrankheiten stabilisiert oder sogar verbessert werden können.

Netzhautdegeneration ist eine wesentliche Ursache für Erblindungen in den Industriestaaten. Verschiedene Untersuchungen geben Hinweise darauf, dass durch geringe elektrische Ströme, die durch die Netzhaut fließen, die erbliche, altersbedingte oder plötzlich auftretende Degeneration der Netzhaut verzögert werden kann.

Dies eröffnet die Möglichkeit, durch regelmäßige, also bspw. tägliche oder wöchentliche, elektrische Stimulation des Auges den allmählichen Verlust des Sehvermögens verzögern zu können, um so betroffenen Patienten länger Teile Ihres Augenlichtes zu erhalten oder sogar Teile des Augenlichtes wiederzugeben.

Diese Behandlung sollte zweckmäßigerweise vom Patienten selbst und zu Hause durchgeführt werden. Dabei muss im Extremfall angenommen werden, dass der Patient praktisch blind ist. Hieraus ergeben sich wesentliche Forderungen an die eingangs erwähnte Vorrichtung sowie die Stimulationselektrode.

Die drahtförmige Stimulationselektrode muss nämlich nach jeder Benutzung, zumindest aber sehr häufig ausgewechselt werden. Zum einen handelt es sich bei der drahtförmigen Stimulationselektrode um eine sehr dünne Elektrode, die bei unsachgemäßer oder auch nach häufiger Handhabung zerbrechen kann.

Zum anderen können es hygienische Erwägungen erforderlich machen, die Stimulationselektrode nach jeder Anwendung auszutauschen.

Die eingangs erwähnte WO 2011/086150 A2 beschreibt für diese Anwendungen drei verschiedene Ausführungsbeispiele.

In einem ersten Ausführungsbeispiel ist die Vorrichtung zur Elektrostimulation des Auges nach Art einer Brille ausgebildet, wobei an den Rahmen, die üblicherweise für die Gläser vorgesehen sind, dann spiralförmige Elektrodenhalter vorgesehen sind, die sich auf das Auge zu erstrecken. Für jedes Auge sind zwei derartige Elektrodenhalter vorgesehen, so dass eine Drahtelektrode mit den beiden inneren Enden dieser nachgiebigen Elektrodenhalter verbunden werden muss, um beim Aufsetzen der Brille dann in Kontakt mit der Cornea zu gelangen.

In einem zweiten Ausführungsbeispiel ist eine Gesichtsmaske gezeigt, die flächig an Teile des Gesichtes des Patienten angepasst ist. In dieser Gesichtsmaske sind Augenöffnungen vorgesehen, an denen Druckknöpfe vorgesehen sind, in die die drahtförmigen Stimulationselektroden eingehakt werden.

In einem dritten Ausführungsbeispiel ist eine Art "Schießbrille" gezeigt, bei der das brillenartige Gestell einen Nasenbügel und zwei davon abgehende Augenbügel umfasst, die wiederum mit klappbaren Seitenbügeln verbunden sind.

An den Augenbügeln sind jeweils zwei Elektrodenhalter befestigt, die längs des Augenbügels sowie quer zu dem Augenbügel, also in allen drei Richtungen verstellbar sind.

An ihren inneren freien Enden sind die Elektrodenhalter jeweils mit Ösen verbunden, in die die drahtförmige Stimulationselektrode eingelegt wird. Mit ihrem ersten Ende wird die Stimulationselektrode an dem nasenseitigen Elektrodenhalter befestigt und dann durch eine Öse in dem schläfenseitigen Elektrodenhalter geleitet. An ihrem zweiten Ende ist die Stimulationselektrode mit einem Gewichtskörper beschwert, so dass die Stimulationselektrode auf diese Weise vorgespannt wird.

Durch einen Fachmann wird dieses Gestell an den Kopf des Patienten angepasst, so dass sich beim Aufsetzen einer derartigen Brille, die mit einer oder zwei Stimulationselektroden bestückt ist, die Stimulationselektrode automatisch an das Auge anlegt. Eine weitere Vorrichtung, welche eine flexible Anpassung mit vielen Freiheitsgraden ermöglichen soll, ist in der WO 2015/104313 A1 beschrieben. Die Vorrichtung wird nach dem einmaligen Einstellen mit einer Klemmschraube als Fixiervorrichtung fest fixiert.

Während das einmalige Einstellen der geometrischen Bedingungen des Gestells durch einen Fachmann erfolgt, soll der Patient anschließend die Vorrichtung zu Hause verwenden, um seine Augen regelmäßig elektrisch zu stimulieren.

Das dazu erforderliche Einhängen der Stimulationselektrode in die Ösen oder an die Elektrodenhalter gestaltet sich für Patienten mit Sehbehinderung jedoch extrem schwierig. Der dünne Elektrodenfaden wird teilweise von den Patienten nicht erkannt oder kann beim Aufsetzen des Brillengestells aus den Elektrodenhaltern herausgleiten.

Um dies zu verhindern, muss die Stimulationselektrode an dem nasenseitigen Elektrodenhalter mehrmals um den Halter gewickelt werden. Dies erfordert großes Geschick, weil der Faden zum einen starr ist und zum anderen die Patienten wegen ihrer Sehbehinderung auf ihr Tastgefühl angewiesen sind.

Vor diesem Hintergrund offenbart die DE 10 2011 055 844 B4 eine weiter verbesserte Stimulationselektrodenanordnung für eine Vorrichtung zur Elektrostimulation des Auges. Die Vorrichtung weist ein brillenartiges Gestell mit einem Nasenteil, eine mit dem Nasenteil verbundene Anordnung zum Halten des Gestells an dem Kopf des Patienten sowie zumindest einen justierbar an dem Nasenteil angeordneten Elektrodenhalter für die drahtförmige Stimulationselektrode auf. Die Stimulationselektrodenanordnung umfasst einen lösbar an dem Elektrodenhalter befestigbaren Elektrodenträger, an dem die Stimulationselektrode angeordnet ist. Der Elektrodenträger ist in einer Ausgestaltung als U-förmiger Bügel mit zwei Armen und einer nach außen offenen Nut ausgebildet. Mit der in der DE 10 2011 055 844 B4 beschriebenen Lösung wird eine Stimulationselektrodenanordnung der eingangs genannten Art bereitgestellt, welche einfach handzuhaben ist und auch sehbehinderten Patienten die Handhabung erleichtert.

Bei der in der DE 10 2011 055 844 B4 beschriebenen Lösung soll der Patient lediglich die Stimulationselektrodenanordnung an dem Elektrodenträger erfassen, der sehr viel massiver ist als die dünne Stimulationselektrode, und diesen Elektrodenträger in den bereits an der Vorrichtung vorhandenen und justierten Elektrodenhalter anbringen.
Weil der Elektrodenträger eine größere Masse und größere Abmaße aufweist als die Stimulationselektrode selbst, können auch sehbehinderte oder gar blinde Patienten den Elektrodenträger ertasten und lagerichtig in den Elektrodenhalter einsetzen.

Ferner offenbart WO 2015/104313 A1 eine medizinische Vorrichtung zum Übertragen von elektrischem Strom auf ein Auge. Die Vorrichtung umfasst gemäß der Zusammenfassung: eine Nasenstütze zum Lagern der Vorrichtung auf der Nase des Patienten, zumindest einen gewinkelt zur Nasenstütze angeordneten und mit der Nasenstütze verbundenen Augensteg und zumindest eine an dem Augensteg befestigte Elektrodenhalterung für eine Augenelektrode zum Kontakt mit einem Auge und zum Übertragen von elektrischem Strom auf ein Auge, wobei die Elektrodenhalterung zumindest zwei Abschnitte aufweist und wobei zumindest ein Abschnitt der Elektrodenhalterung mittels zumindest eines Gelenks relativ zu dem Augensteg und/oder zu einem anderen Abschnitt der Elektrodenhalterung bewegbar ausgebildet ist. Ferner offenbart US 2023/082686 A1 eine tragbare medizinische Vorrichtung. US 2018/318586 A1 offenbart ein direktes elektrisches Stimulationsbereitstellungssystem zur Behandlung von Augenerkrankungen. US 2022/0305265 A1 offenbart eine kopfgetragene Vorrichtungen für die Augenbehandlung.

Vor diesem Hintergrund liegt der vorliegenden Erfindung die Aufgabe zugrunde, eine Vorrichtung, insbesondere zur Elektrostimulation des Auges, bereitzustellen, welche die Handhabung weiter verbessert und ein angenehmeres Nutzererlebnis ermöglicht. Insbesondere wäre es wünschenswert eine Applikation eines in Eingriff eines mit dem Auge des Nutzers zu bringenden Elements zu erleichtern.

Die beanspruchten Gegenstände sind in den unabhängigen Ansprüchen definiert. Vorteilhafte Weiterbildungen sind in den abhängigen Ansprüchen beschrieben.
Gemäß einem ersten Aspekt der vorliegenden Offenbarung wird daher vorgeschlagen, eine Vorrichtung, insbesondere eine Vorrichtung zur Elektrostimulation des Auges, bereitzustellen, wobei die Vorrichtung ein brillenartiges Gestell mit einem Nasenteil, eine mit dem Nasenteil verbundene Anordnung zum Halten des Gestells an dem Kopf des Nutzers sowie zumindest einen an dem Gestell angeordneten Halter für ein in Eingriff mit einem Auge des Nutzers zu bringendes Element, insbesondere für eine drahtförmige Stimulationselektrode, aufweist, wobei der Halter dazu eingerichtet ist (relativ zum Gestell), zwischen einer Nicht-Eingriffsposition und einer vordefinierten Eingriffsposition hin und her bewegt zu werden. Der Halter kann einen nasalen vertikalen Schenkel und einen temporalen vertikalen Schenkel aufweisen, welche sich von dem Halter, insbesondere von einem horizontalen Quersteg des Halters, in vertikaler Richtung nach unten erstrecken, wobei der nasale vertikale Schenkel und/oder der temporale vertikale Schenkel längenverstellbar sind. Alternativ oder zusätzlich kann der Halter einen nasalen horizontalen Schenkel und einen temporalen horizontalen Schenkel aufweisen, wobei der nasale horizontale Schenkel eine erste Aufnahme für das in Eingriff mit dem Auge des Nutzers zu bringende Element aufweist, wobei der temporale horizontale Schenkel eine zweite Aufnahme für das in Eingriff mit dem Auge des Nutzers zu bringende Element aufweist, und wobei der nasale horizontale Schenkel und/oder der temporale horizontale Schenkel längenverstellbar sind.

Die Erfinder haben erkannt, dass bei herkömmlichen Vorrichtungen zur Elektrostimulation des Auges in der Regel zwei Personen eingebunden sind, um die mit den Stimulationselektroden bestückte Brille an die Augen anzulegen. Während eine Hilfsperson dem Nutzer die Brille mit den Stimulationselektroden aufsetzt und beim Aufsetzen der Brille gleichzeitig die Stimulationselektroden auf die Augen auflegt, hält der Nutzer mit seinen Händen beide Augen offen und sorgt dafür, dass die Stimulationselektroden nicht mit den Wimpern kollidieren.

Die Erfinder haben ferner erkannt, dass es beim Aufsetzen der Brille einer herkömmlichen Vorrichtung zur Elektrostimulation, insbesondere wenn Ohrenbügel der Brille hinter die Ohren des Nutzers eingefädelt werden, zu einer Art geringfügigen Sägebewegung kommen kann, d.h. zu einer Bewegung, bei welcher die Stimulationselektroden in Längsrichtung der Stimulationselektroden über die Augen des Nutzers gezogen werden, was von Nutzern als unangenehm empfunden werden kann.

Es wird daher vorgeschlagen, das in Eingriff mit dem Auge des Nutzers zu bringende Element, wie beispielsweise eine Stimulationselektrode oder Kontaktlinse, nicht gegenüber dem brillenartigen Gestell der Vorrichtung vollständig fixiert für den Nutzer bereitzustellen. Stattdessen wird vorgeschlagen, einen an dem Gestell angeordneten Halter bereitzustellen, wobei der Halter dazu eingerichtet ist, relativ zum Gestell hin und her bewegt zu werden und zwar zwischen einer Nicht-Eingriffsposition und einer vordefinierten Eingriffsposition.

Wie eingangs beschrieben ist bei herkömmlichen Vorrichtungen zur Elektrostimulation kein derartig eingerichteter Halter vorgesehen. Allenfalls erfolgt einmalig eine Einstellung an die Anatomie des Nutzers. Die Einstellung wird beispielsweise von einem Augenarzt festgelegt. Bei nachfolgenden Anwendungen wird die voreingestellte Vorrichtung mit fest justierten Elektrodenhaltern vom Nutzer oder einer Hilfsperson aufgesetzt, d.h. gelangen die Elektroden beim Aufsetzen der Brille in Kontakt mit der Cornea.

Demgegenüber kann die vorgeschlagene Vorrichtung zunächst aufgesetzt werden, wobei sich der Halter noch in der Nicht-Eingriffsposition befindet. In der Nicht-Eingriffsposition ist das in Eingriff mit dem Auge des Nutzers zu bringende Element allerdings selbst bei aufgesetzter Vorrichtung noch nicht in Kontakt bzw. in Eingriff mit dem Auge des Nutzers. Erst nach dem Aufsetzen wird der Halter von der Nicht-Eingriffsposition in die vordefinierte Eingriffsposition bewegt. In der vordefinierten Eingriffsposition ist das in Eingriff mit dem Auge des Nutzers zu bringende Element bei aufgesetzter Vorrichtung in einer vordefinierten Position in Eingriff mit dem Auge des Nutzers. Statt auf beliebige Art und Weise Kontakt mit dem Auge des Nutzers herzustellen, ist die Eingriffsposition der Vorrichtung vordefiniert und der Halter ist spezifisch dazu eingerichtet zu dieser vordefinierten Eingriffsposition bewegt zu werden.

Die vordefinierte Eingriffsposition kann beispielsweise von einem Experten, beispielsweise einem Augenarzt, angepasst werden. Beispielsweise kann ein Anschlag, an welchem der Halter in der vordefinierten Eingriffsposition anliegen kann, eingestellt werden. Bei der nachfolgenden Anwendung kann der Nutzer selbst den Halter zwischen der Nicht-Eingriffsposition zum Aufsetzen der Vorrichtung und der vordefinierten Eingriffsposition hin und her bewegen. Mit anderen Worten kann unter einer vordefinierten Eingriffsposition im Sinne der vorliegenden Offenbarung eine vorgegebene bzw. festlegbare bzw. voreinstellbare Eingriffsposition verstanden werden. Insbesondere kann die Vorrichtung einen, insbesondere einstellbaren, Anschlag (eine Begrenzungseinrichtung) aufweisen, welcher dazu eingerichtet ist, in der vordefinierten Eingriffsposition eine Bewegung des Halters in einer Raumrichtung zu begrenzen. Es ist jedoch auch möglich, dass das Einstellen der Eingriffsposition durch Einstellen von einem oder mehreren Elementen des Halters erfolgen kann, welcher in der Eingriffsposition an einen nicht einstellbaren vorgegebenen Anschlag anliegen kann. Die Vorrichtung kann dazu eingerichtet sein in der vordefinierten Eingriffsposition eine Bewegung des Halters über die vordefinierte Eingriffsposition hinaus in Richtung des Auges zu begrenzen bzw. zu verhindern. Die Vorrichtung ist jedoch weiterhin dazu eingerichtet, eine Bewegung des Halters zwischen der Nicht-Eingriffsposition und der vordefinierten Eingriffsposition zu ermöglichen. Ein Vorteil dieser Lösung kann darin bestehen, dass die Handhabung für sehbehinderte Nutzer weiter verbessert und ein angenehmeres Nutzererlebnis ermöglicht werden kann.

Indem die Vorrichtung dazu eingerichtet ist, eine vordefinierte Eingriffsposition bereitzustellen und die Vorrichtung dazu eingerichtet ist, nicht nur beliebig bewegt zu werden, sondern dazu eingerichtet ist zwischen einer Nicht-Eingriffsposition und der vordefinierten Eingriffsposition hin und her bewegt zu werden, ist eine reproduzierbare Positionierung des in Eingriff mit dem Auge des Nutzers zu bringenden Elements, insbesondere einer Stimulationselektrode auf der Augenoberfläche, in der Eingriffsposition möglich. Insbesondere wird eine reproduzierbare Positionierung auch durch einen ungeübten Nutzer ermöglicht.

Die Vorrichtung kann eine Vorrichtung zur Elektrostimulation des Auges sein. Der Halter kann ein Elektrodenhalter für eine (drahtförmige) Stimulationselektrode bzw. Stimulationselektrodenanordnung sein. Die Stimulationselektrodenanordnung kann einen ersten Elektrodenträger und einen zweiten Elektrodenträger aufweisen, wobei die Stimulationselektrode zwischen dem ersten Elektrodenträger und dem zweiten Elektrodenträger angeordnet ist, wobei der erste und/oder zweite Elektrodenträger dazu eingerichtet ist, magnetisch an dem Elektrodenhalter befestigt zu werden.

Die Erfinder haben erkannt, dass die in den eingangs genannten eigenen früheren Anmeldungen beschriebenen Lösungen die Handhabung einer Stimulationselektrode zur Elektrostimulation des Auges für sehbehinderte Nutzer bereits deutlich verbessern. Die Erfinder haben jedoch erkannt, dass das zielgenaue Platzieren der Stimulationselektrodenanordnung am Elektrodenhalter für sehbehinderte Nutzer weiterhin Schwierigkeiten bereitet. Gemäß diesem Aspekt wird daher vorgeschlagen, dass die Stimulationselektrodenanordnung einen ersten Elektrodenträger und einen zweiten Elektrodenträger aufweist, wobei die Stimulationselektrode zwischen dem ersten Elektrodenträger und dem zweiten Elektrodenträger angeordnet ist, wobei der erste und/oder zweite Elektrodenträger dazu eingerichtet ist, magnetisch an dem Elektrodenhalter befestigt zu werden.

Indem der Elektrodenträger magnetisch an dem Elektrodenhalter befestigt wird, wird die Handhabung für sehbehinderter Nutzer weiter erleichtert. Elektrodenträger und Elektrodenhalter ziehen einander magnetisch an, sodass eine gewisse Selbstpositionierung von Elektrodenträger und Elektrodenhalter relativ zueinander erfolgen kann. Eine ungefähre bzw. nicht perfekte Positionierung des Elektrodenhalters, wie diese auch von einem sehbehinderten Nutzer erreicht werden kann, kann daher ausreichen, um den Elektrodenträger magnetisch aufzunehmen.

Ein weiterer Vorteil dieser Ausgestaltung kann darin bestehen, dass die Hygiene bei der Handhabung einer Stimulationselektrodenanordnung für eine Vorrichtung zur Elektrostimulation des Auges verbessert werden kann. Die Erfinder haben erkannt, dass durch den magnetisch am Elektrodenhalter befestigbaren Elektrodenträger, weniger Versuche des sehbehinderten Nutzers zur korrekten Befestigung des Elektrodenträgers am Elektrodenträger erforderlich sein können. Hierdurch kann das Risiko, dass bei unsachgemäßer Handhabung Verunreinigungen in das Auge eingebracht werden, reduziert und die Hygiene bei der Handhabung weiter verbessert werden.

Im Rahmen der vorliegenden Offenbarung kann unter einer Stimulationselektrodenanordnung eine Elektrodenanordnung zum Stimulieren und/oder Messen verstanden werden. Entsprechend kann unter einer Stimulationselektrode eine Elektrode zum Stimulieren und/oder Messen verstanden werden. Mit anderen Worten kann nicht nur ein elektrisches Stimulationssignal abgegeben werden, sondern auch ein Messsignal erfasst werden. Beispielsweise kann mit der Stimulationselektrode bzw. Stimulationselektrodenanordnung ein Stimulus appliziert werden oder ein Elektroretinogramm bzw. ERG-Signal erfasst werden. Entsprechend kann im Rahmen der vorliegenden Offenbarung unter einer Vorrichtung zur Elektrostimulation des Auges eine Vorrichtung zum Stimulieren und/oder Messen verstanden werden. Entsprechend kann im Rahmen der vorliegenden Offenbarung unter einem System zur Elektrostimulation des Auges ein System zum Stimulieren und/oder Messen verstanden werden.

Die Vorrichtung kann zwei separate Halter aufweisen, einen linken Halter für ein in Eingriff mit einem linken Auge des Nutzers zu bringendes Element, insbesondere für eine Stimulationselektrode für das linke Auge, und einen rechten Halter für ein in Eingriff mit einem rechten Auge des Nutzers zu bringendes Element, insbesondere für eine Stimulationselektrode für das rechte Auge. Ein Vorteil kann darin bestehen, dass die Handhabung weiter verbessert wird.

In einer Weiterbildung können der linke Halter und der rechte Halter dazu eingerichtet sein, separat voneinander zwischen einer jeweiligen Nicht-Eingriffsposition und einer jeweiligen Eingriffsposition hin und her bewegt zu werden. Mit anderen Worten kann der linke Halter dazu eingerichtet sein, unabhängig vom rechten Haltern zwischen einer Nicht-Eingriffsposition für den linken Halter und einer Eingriffsposition für den linken Halter, in welcher ein in Eingriff mit dem linken Auge des Nutzers zu bringendes Element in Eingriff mit dem linken Auge des Nutzers gebracht werden kann, hin und her bewegt zu werden. Entsprechend kann der rechte Halter dazu eingerichtet sein, unabhängig vom linken Haltern zwischen einer Nicht-Eingriffsposition für den rechten Halter und einer Eingriffsposition für den rechten Halter, in welcher ein in Eingriff mit dem rechten Auge des Nutzers zu bringendes Element in Eingriff mit dem rechten Auge des Nutzers gebracht werden kann, hin und her bewegt zu werden. Hierdurch kann die Handhabung weiter vereinfacht werden. Beispielsweise kann der Nutzer selbst mit einer Hand das Auge aufhalten und mit der anderen Hand den korrespondierenden Halter für das Auge von der Nicht-Eingriffsposition in die Eingriffsposition bewegen.

Der Halter kann dazu eingerichtet sein, zwischen einer einstellbaren vordefinierten Nicht-Eingriffsposition und/oder einer einstellbaren vordefinierten Eingriffsposition hin und her bewegt zu werden. Mit anderen Worten können die Nicht-Eingriffsposition und die Eingriffsposition (Behandlungsposition) vordefiniert sein. Die Vorrichtung mit dem Halter ist dazu eingerichtet, dass eine Bewegung zwar möglich ist, aber nur bis zu der vordefinierten Eingriffsposition. Hierbei kann die vordefinierte Eingriffsposition, bis zu welcher eine Bewegung, insbesondere in Richtung des Auges entgegen der Blickrichtung, erfolgen kann, einstellbar sein und individuell für den Nutzer angepasst werden. Die Einstellung der Eingriffsposition kann hierbei durch eine Einstellung der Bewegung des Halters, beispielsweise durch Einstellen eines Anschlags bzw. einer Begrenzungseinrichtung erfolgen. Es ist jedoch auch möglich, dass die Einstellung der Eingriffsposition durch Einstellen von einem oder mehreren Elementen des Halters erfolgt, beispielsweise durch Anpassung einer Länge und/oder eines Abstandes. Somit sind selbst bei einem vorgegebenen Anschlag verschiedene Positionen möglich, in welchen das in Eingriff mit dem Auge des Nutzers zu bringende Element in der Eingriffsposition in Eingriff mit dem Auge des Nutzers gebracht werden kann. Optional kann die Nicht-Eingriffsposition ebenfalls vordefiniert sein. Beispielsweise kann der Halter dazu eingerichtet sein, in einer vordefinierten Nicht-Eingriffsposition einzurasten bzw. lösbar arretiert zu werden. Dies erleichtert die Handhabung in einem definierten Zustand, ohne dass es zu einer unbeabsichtigten Bewegung, wie einem unbeabsichtigten zu schnellen Auftreffen des in Eingriff mit dem Auge des Nutzers zu bringenden Elements auf dem Auge des Nutzers, kommt.

Die Vorrichtung kann dazu eingerichtet sein, dass in der vordefinierte Eingriffsposition eine Bewegung des Halters in Richtung des Auges des Nutzers mechanisch begrenzt ist, insbesondere wobei die Begrenzung einstellbar sein kann. Mit anderen Worten kann in der Eingriffsposition (Behandlungsposition) eine weitere Bewegung über die Begrenzung hinaus mechanisch verhindert werden, insbesondere in Richtung des Auges entgegen der Blickrichtung. Beispielsweise kann damit vermieden werden, dass das in Eingriff mit dem Auge des Nutzers zu bringende Element einen zu großen Druck auf das Auge ausübt. Die Begrenzung kann eine einstellbare Begrenzung sein, welche für den Nutzer angepasst werden kann, um eine gewünschte vordefinierte Eingriffsposition bereitzustellen. Es ist jedoch auch möglich, dass die Einstellung der Eingriffsposition durch Einstellen von einem oder mehreren Elementen des Halters erfolgt, welcher in der Eingriffsposition an einen nicht einstellbaren vorgegebenen Anschlag bzw. eine Begrenzungseinrichtung anliegen kann.

Die Vorrichtung mit dem Halter kann dazu eingerichtet sein, dass der Halter in der vordefinierten Eingriffsposition an einen von dem Gestell bereitgestellten Endanschlag anliegt. Mit anderen Worten kann in der Eingriffsposition (Behandlungsposition) eine weitere Bewegung über die Eingriffsposition hinaus durch den Endanschlag verhindert werden. Unter einem Endanschlag kann eine definierte mechanische Begrenzung verstanden werden, welche optional einstellbar sein kann. Beispielsweise kann damit vermieden werden, dass das in Eingriff mit dem Auge des Nutzers zu bringende Element einen zu großen Druck auf das Auge ausübt. Der Endanschlag kann ein einstellbarer Endanschlag sein, welcher für den Nutzer angepasst werden kann, um eine gewünschte vordefinierte Eingriffsposition bereitzustellen.

Der Halter kann ein schwenkbarer Halter sein, welcher dazu eingerichtet ist, zwischen der Nicht-Eingriffsposition und der vordefinierten Eingriffsposition hin und her geschwenkt zu werden. Ein Vorteil kann darin bestehen, dass eine Schwenkbewegung eine einfache Handhabung bietet und ein angenehmes Auflegen des in Eingriff mit dem Auge des Nutzers zu bringenden Elements ermöglicht.

Ein Radius für die Schwenkbewegung kann vorzugsweise zwischen 1 cm und 7 cm, insbesondere zwischen 2 cm und 5 cm, insbesondere zwischen 2,5 cm und 4 cm betragen. Beispielsweise kann am Halter ein Drehgriff vorgesehen sein, mit welchem der Halter zwischen der Nicht-Eingriffsposition und der Eingriffsposition hin und her geschwenkt werden kann. Mit den vorstehend genannten Radien besteht ein vorteilhafter Zusammenhang zwischen einer Winkelgeschwindigkeit der Schwenkbewegung, mit welcher Drehgriff betätigt werden kann, und einer Geschwindigkeit, mit welcher das Element in Kontakt mit dem Auge des Nutzers gebracht wird. Bei einer zu langsamen Bewegung besteht ein Risiko, dass der Nutzer während des In-Eingriff-Bringens blinzelt. Bei einer zu schnellen Bewegung besteht hingegen das Risiko, das das In-Eingriff-Bringen als unangenehm empfunden wird.

Die Vorrichtung mit dem Halter kann dazu eingerichtet sein, dass der Halter über mindestens eine vordefinierte Zwischenposition, welche sich zwischen der Nicht-Eingriffsposition und der Eingriffsposition befindet, zwischen der Nicht-Eingriffsposition und der Eingriffsposition hin und her bewegt werden kann. Insbesondere kann eine Rasteinrichtung vorgesehen sein, um den Halter in der vordefinierten Zwischenposition zu halten. Beispielsweise kann das in Eingriff mit dem Auge des Nutzers zu bringende Element mit der vordefinierten Zwischenposition bereits in einem ersten Schritt in die Nähe des Auges gebracht werden und erst in einem weiteren Schritt in Eingriff gebracht werden. Dies kann die Handhabung insbesondere bei sehbehinderten Nutzern weiter erleichtern.

Der Halter kann dazu eingerichtet sein, das in Eingriff mit dem Auge des Nutzers zu bringende Element mittels einer Drehbewegung in die Eingriffsposition zu bewegen, wobei eine Drehachse für die Drehbewegung eine vertikale Achse ist. Unter einer vertikalen Achse ist hierbei eine vertikale Achse bezogen auf eine Trageposition der Vorrichtung auf dem Kopf des Nutzers zu verstehen. Ein Vorteil dieser Ausgestaltung kann darin bestehen, dass der Halter beispielsweise durch ein seitliches Einschwenken in die Eingriffsposition bewegt werden kann und minimale Hebelkräfte auf das Auge wirken.

Der kann Halter dazu eingerichtet ist, das in Eingriff mit dem Auge des Nutzers zu bringende Element mittels einer Drehbewegung in die Eingriffsposition zu bewegen, wobei eine Drehachse für die Drehbewegung eine horizontale Achse ist. Unter einer horizontalen Achse ist hierbei eine horizontale Achse bezogen auf eine Trageposition der Vorrichtung auf dem Kopf des Nutzers zu verstehen. Ein Vorteil dieser Ausgestaltung kann darin bestehen, dass das in Eingriff mit dem Auge des Nutzers zu bringende Element beispielsweise von vorne (entgegengesetzt der in Blickrichtung) insbesondere von vorne schräg unten in Eingriff mit dem Auge des Nutzers gebracht werden kann. Dies kann das Einführen durch geöffnete Lieder erleichtern, ohne an den Wimpern hängen zu bleiben.

In einer Weiterbildung kann die horizontale Achse in vertikaler Richtung oberhalb der Augen des Nutzers liegen. Der Halter kann dazu eingerichtet sein, das in Eingriff mit dem Auge des Nutzers zu bringende Element waagrecht auf das Auge des Nutzers aufzusetzen. Der Halter kann dazu eingerichtet sein, dass in Eingriff mit dem Auge des Nutzers zu bringende Element mit einer exzentrischen Schwenkbewegung auf das Auge des Nutzers aufzusetzen bzw. mit diesem in Eingriff zu bringen. Dies ermöglicht eine einfache Handhabung und gleichzeitig eine vorteilhafte Trajektorie für das in Eingriff mit dem Auge des Nutzers zu bringende Element in die Eingriffsposition.

Der Halter kann einen Betätigungsgriff zur Bewegung des Halters zwischen der Nicht-Eingriffsposition und der Eingriffsposition aufweisen. Insbesondere kann der Betätigungsgriff in axialer Richtung entlang der Drehachse angeordnet sein. Ein Vorteil dieser Ausgestaltung kann in einer einfachen Handhabung bestehen. Der Betätigungsgriff kann ein Drehgriff sein. Ein Vorteil dieser Ausgestaltung kann darin bestehen, dass ein Betätigungsgriff in Form eines Drehgriffs auch mit motorischen Einschränkungen der Finger, wie dies häufig bei älteren Menschen der Fall ist, leicht betätigt werden kann. Hierbei kann die Drehachse für die Drehung in die Eingriffsposition innerhalb des Drehgriffs liegen. Insbesondere kann die Drehachse mit einer Mittelachse des Drehgriffs zusammenfallen. Ein Vorteil dieser Ausgestaltung kann darin bestehen, dass das mit der Drehung am Drehgriff verbundene Drehmoment unabhängig von den Einstellungen oder Abständen der einzelnen Abschnitte der Elektrodenhalter von der Drehachse ist und insbesondere nicht von den Einstellungen der Längen an den Schenkeln für die Elektrodenaufnahmen abhängt. Der Betätigungsgriff kann insbesondere ein Drehgriff sein, welcher in temporaler Richtung neben dem Halter angeordnet ist. Mit anderen Worten kann der Drehgriff in axialer Richtung insbesondere rechts neben einem rechten Halter oder links neben einem linken Halter angeordnet sein. Die Anordnung von Drehgriffen links und/oder rechts kann insbesondere eine einhändige Drehung bei aufgesetzter Vorrichtung ermöglichen. Eine Anordnung des Drehgriffs in Verlängerung der Drehachse kann insbesondere eine unerwünschte Krafteinwirkung auf das Auge vermeiden. Beispielsweise kann eine Hebelwirkung, welche bei einem nicht in axialer Richtung entlang der Drehachse angeordneten Betätigungsgriff auftreten könnte, vermieden oder zumindest reduziert werden.

Der Halter kann einen Betätigungsgriff zur Bewegung des Halters zwischen der Nicht-Eingriffsposition und der Eingriffsposition aufweisen. Der Betätigungsgriff kann (in horizontaler Richtung) über das Gestell der Vorrichtung hinausragen. Ein Vorteil dieser Ausgestaltung kann in einer einfachen Handhabung bestehen. Insbesondere kann ein in horizontaler Richtung über das Gestell hinausragender Betätigungsgriff betätigt werden, ohne das in Eingriff mit dem Auge des Nutzers zu bringende Element zu berühren und ggf. zu verunreinigen. Der Betätigungsgriff, insbesondere ein Drehgriff, kann zumindest teilweise temporal außerhalb des Gestells angeordnet sein. Ein Vorteil kann darin bestehen, dass der Betätigungsgriff nicht das zentrale Gesichtsfeld verdeckt, wenn der Halter in der Eingriffsposition ist.

Der Halter kann einen Betätigungsgriff zur Bewegung des Halters zwischen der Nicht-Eingriffsposition und der Eingriffsposition aufweisen, wobei die Vorrichtung dazu eingerichtet ist, dass der Betätigungsgriff außerhalb eines Gesichtsfelds des Nutzers, insbesondere außerhalb eines zentralen Gesichtsfelds des Nutzers angeordnet ist. Insbesondere kann die Vorrichtung dazu eingerichtet sein, dass der Betätigungsgriff in der Eingriffsposition außerhalb eines zentralen Gesichtsfelds des Nutzers angeordnet ist. Bei dem Betätigungsgriff kann es sich um einen Drehgriff handeln. Der Elektrodenhalter und Betätigungsgriff können so ausgeführt und angeordnet sein, dass insbesondere das zentrale Gesichtsfeld des Nutzers vor allem in der Eingriffsposition und/oder optional in der Nicht-Eingriffsposition frei bleibt. Ein Vorteil dieser Ausgestaltung kann darin bestehen, dass die Handhabung weiter verbessert wird. Insbesondere kann mit der vorgeschlagenen Anordnung außerhalb des Gesichtsfelds die Wahrscheinlichkeit eines unerwünschten Lidschlussreflex beim Aufbringen einer Stimulationselektrode auf ein Auge des Nutzers reduziert werden.

Der Halter kann dazu eingerichtet sein, einhändig und/oder werkzeuglos, zwischen der Nicht-Eingriffsposition und der vordefinierten Eingriffsposition hin und her bewegt zu werden. Mit anderen Worten kann die Vorrichtung mit dem Halter entsprechend eingerichtet sein. Indem die Vorrichtung mit dem Halter dazu eingerichtet ist, dass der Halter werkzeuglos zwischen einer Nicht-Eingriffsposition und einer vordefinierten Eingriffsposition hin und her bewegt werden kann, ist klargestellt, dass es sich um eine Bewegung handelt, welche der Nutzer im Betrieb mehrfach auf einfache Art und Weise durchführen kann. Insbesondere kann die Bewegung vom Nutzer selbst durchgeführt werden, nachdem die Vorrichtung bereits an den Nutzer angepasst wurde, insbesondere nachdem die Vorrichtung bereits auf den Kopf des Nutzers aufgesetzt wurde. Vorzugsweise ist keine Reibschlussverbindung vorgesehen. Indem die Vorrichtung mit dem Halter dazu eingerichtet ist, dass der Halter einhändig zwischen der Nicht-Eingriffsposition und der vordefinierten Eingriffsposition hin und her bewegt werden kann, hat der Nutzer eine weitere Hand frei, um beispielsweise das Auge mit der weiteren Hand offen zu halten. Hierdurch kann die Handhabung insbesondere für einen sehbehinderten Nutzer, welcher die Vorrichtung ohne Hilfspersonen nutzen möchte, weiter verbessert werden.

Die Vorrichtung kann dazu eingerichtet sein, dass Anpassungen aller veränderlichen Teile und deren Arretierung in den ausgewählten Einstellungen ohne Werkzeug durchgeführt werden können. Ein Vorteil kann darin bestehen, dass Anpassungen auf einfache Art und Weise vorgenommen werden können.

Der Halter kann dazu eingerichtet sein, das in Eingriff mit dem Auge des Nutzers zu bringende Element in einem Winkel im Bereich +/- 30°, insbesondere in einem Winkel im Bereich +/-20° zur Waagrechten von vorne auf das Auge des Nutzers in die vordefinierte Eingriffsposition zu bringen. Ein Vorteil dieser Ausgestaltung kann darin bestehen das Einführen durch die Augenlieder vereinfacht wird. Insbesondere kann eine vordefinierte Einschwenkbewegung erfolgen, wobei beim In-Eingriff-Bringen vorzugsweise kein "Verdrehen" bzw. keine Sägebewegung des Elements auf dem Auge erfolgt.

Der Halter kann einen horizontalen Quersteg aufweisen, welcher in horizontaler Richtung verstellbar an dem Gestell angeordnet ist.

Ein Bedienelement, um den Halter in horizontaler Richtung gegenüber dem Gestell zu verstellen, kann in dem Betätigungsgriff angeordnet sein. Mit anderen Worten können der Betätigungsgriff zur Bewegung des Halters zwischen der Nicht-Eingriffsposition und der Eingriffsposition zusammen angeordnet sein, wobei das Bedienelement in dem Betätigungsgriff angeordnet ist. Dieser Aufbau kann eine koaxiale Anordnung von Betätigungsgriff und Bedienelement ermöglichen. Beispielsweise kann eine Achse für eine horizontale Schwenkbewegung mit einer Achse, in deren Längsrichtung eine Verstellung des Halters in horizontaler Richtung gegenüber dem Gestell erfolgt, zusammenfallen. Dies kann einen kompakten Aufbau ermöglichen. Außerdem kann diese Anordnung mit gleichen Achsen eine intuitive Bedienung ermöglichen. Es kann also ausreichen, dass pro Elektrodenhalter nur eine gemeinsame Anordnung umfassend Bedienelement und Betätigungsgriff bereitgestellt wird. Insbesondere für sehbehinderte Nutzer kann die Bedienung mit der vorgeschlagenen Anordnung erleichtert werden. Die zusammen angeordneten Bedienelemente könnten auf einfache Art und Weise taktil erfasst und betätigt werden.

Der Halter kann einen nasalen vertikalen Schenkel und einen temporalen vertikalen Schenkel aufweisen, welche sich von dem Halter, insbesondere von einem horizontalen Quersteg des Halters, in vertikaler Richtung nach unten erstrecken. Der nasale vertikale Schenkel und/oder der temporale vertikale Schenkel können optional längenverstellbar sein. Die Längenverstellung eines oder beider der Schenkel kann somit eine Höhenanpassung des in Eingriff mit einem Auge des Nutzers zu bringenden Elements gegenüber dem Auge des Nutzers ermöglichen.

Der Halter kann einen nasalen horizontalen Schenkel und einen temporalen horizontalen Schenkel aufweisen, wobei der nasale horizontale Schenkel eine erste Aufnahme für das in Eingriff mit dem Auge des Nutzers zu bringende Element aufweist, wobei der temporale horizontale Schenkel eine zweite Aufnahme für das in Eingriff mit dem Auge des Nutzers zu bringende Element aufweist. Der nasale horizontale Schenkel und/oder der temporale horizontale Schenkel können optional längenverstellbar sein. Die Längenverstellung eines oder beider der Schenkel kann somit eine Abstandsanpassung des in Eingriff mit einem Auge des Nutzers zu bringenden Elements gegenüber dem Auge des Nutzers ermöglichen. Dank der oder den Längenanpassungen kann somit auf einfache Weise angepasst werden, wo das in Eingriff mit dem Auge des Nutzers zu bringende Element zum Liegen kommt, wenn sich der Halter in der vordefinierten Eingriffsposition befindet.

Die erste Aufnahme des nasalen horizontalen Schenkels und die zweite Aufnahme des temporalen horizontalen Schenkels können einen festen horizontalen Abstand voneinander aufweisen. Ein Vorteil dieser spezifischen Ausgestaltung kann darin bestehen, dass ein von den beiden Aufnahmen aufzunehmendes Element nicht für unterschiedliche Abstände angepasst werden muss.

Beispielsweise kann es sich bei dem in Eingriff mit einem Auge des Nutzers zu bringenden Element um eine Stimulationselektrodenanordnung handeln. Die Stimulationselektrodenanordnung kann einen ersten Elektrodenträger und einen zweiten Elektrodenträger aufweisen, wobei die Stimulationselektrode zwischen dem ersten Elektrodenträger und dem zweiten Elektrodenträger angeordnet ist. Die Stimulationselektrodenanordnung kann hierbei in einer Blisterpackung bereitgestellt werden, in welcher der erste und zweite Elektrodenträger in einem definierten Abstand voneinander angeordnet sind, wobei der definierte Abstand dem festen horizontalen Abstand der ersten Aufnahme des nasalen horizontalen Schenkels und der zweiten Aufnahme des temporalen horizontalen Schenkels entspricht. Obwohl der Halter anpassbar ist, ermöglicht der feste Abstand der Aufnahmen eine einfache Entnahme direkt aus einer vorgegebenen Anordnung, in welcher die aufzunehmenden Elemente ebenfalls in dem festen vorgegebenen Abstand bereitgestellt sind. Ein Aufwand für die Lagerhaltung von Stimulationselektrodenanordnungen kann damit reduziert werden. Ferner kann die Hygiene verbessert werden, da die Stimulationselektrode nicht von Hand entnommen und der Abstand der beiden Elektrodenträger nicht von Hand angepasst werden muss.

In einer vorteilhaften Ausgestaltung können sich der nasale vertikale Schenkel und der temporale vertikale Schenkel von dem horizontalen Quersteg des Halters in vertikaler Richtung nach unten erstrecken. Der nasale horizontale Schenkel kann an einem unteren Ende des nasalen vertikalen Schenkels angeordnet sein. Der temporale horizontale Schenkel kann an einem unteren Ende des temporalen vertikalen Schenkels angeordnet sein. Insbesondere können die Komponenten derart angeordnet sein, dass in Blickrichtung des Nutzers ein freier optischer Pfad bereitgestellt wird.

In einer Weiterbildung können der nasale vertikale Schenkel und der temporale vertikale Schenkel orthogonal zum Quersteg des Halters angeordnet sein. Der nasale horizontale Schenkel und der temporale horizontale Schenkel können orthogonal zum Quersteg des Halters angeordnet sein. Der nasale horizontale Schenkel kann orthogonal zum nasalen vertikalen Schenkel angeordnet sein. Der temporale horizontale Schenkel kann orthogonal zum temporalen vertikalen Schenkel angeordnet sein. Ein Vorteil dieser Anordnung kann darin bestehen, dass die Vorrichtung flexibel und auf einfache Weise mit definiert zueinander ausgerichteten Elementen an den Nutzer anpassbar ist. Ferner kann für den Nutzer, auch wenn sich der Halter in der vordefinierten Eingriffsposition befindet ein freier optischer Pfad bereitgestellt werden. Dies kann einen empfundenen Tragekopfort bei einer Behandlung, beispielsweise bei der Elektrostimulation des Auges, verbessern.

Der nasale vertikale Schenkel und/oder der temporale vertikale Schenkel und/oder der nasale horizontale Schenkel und/oder der temporale horizontale Schenkel können jeweils mit einer Gewindespindel längenverstellbar sein. Beispielsweise kann jeweils ein Drehgriff zur Betätigung der Gewindespindel vorgesehen sein. In der vorliegenden Anwendung ist damit eine akkurate Einstellung der gewünschten Länge möglich.

Der Halter kann dazu eingerichtet sein, das in Eingriff mit dem Auge des Nutzers zu bringende Element an einem ersten Auflagepunkt und einem zweiten Auflagepunkt in der Eingriffsposition in Eingriff mit dem Auge des Nutzers zu bringen. Der Halter kann dazu eingerichtet sein, den ersten Auflagepunkt in drei Raumrichtungen zu justieren und den zweiten Auflagepunkt in drei Raumrichtungen zu justieren. Allerdings kann vorzugsweise genau eine Raumrichtung derart gekoppelt sind, das der erste und zweite Auflagepunkt einen definierten Abstand in dieser Raumrichtung aufweisen. Ein Vorteil dieser Lösung kann darin bestehen, dass zuverlässig reproduzierbare Auflagepunkte bereitgestellt werden können. Dabei können zwei Auflagepunkte je Auge bereitgestellt werden, die jeweils in drei Koordinatenachsen an den Nutzer angepasst sein können. Ein Abstand kann vorteilhaft fest definiert sein, um beispielsweise ein in Eingriff mit dem Auge des Nutzers zu bringendes Element mit einem definierten Abstand aufnehmen zu können, beispielsweise aus einer Verpackung, insbesondere einer Blisterpackung, wie beispielhaft hierin beschrieben.

Es sind verschiedene in Eingriff mit dem Auge des Nutzers zu bringende Elemente denkbar. Im Rahmen der vorliegenden Offenbarung kann unter einem ein in Eingriff mit einem Auge des Nutzers zu bringenden Element auch ein auf einem Auge des Nutzers zu applizierendes Element verstanden werden. Wie eingangs erwähnt kann das in Eingriff mit dem Auge des Nutzers zu bringende Element eine Stimulationselektrode bzw. Stimulationselektrodenanordnung sein. Das in Eingriff mit dem Auge des Nutzers zu bringende Element kann auch eine Kontaktlinse sein; wobei der Halter ein Halter für die Kontaktlinse ist und dazu eingerichtet ist, die Kontaktlinse in der vordefinierten Eingriffsposition auf das Auge des Nutzers zu applizieren.

Ferner betrifft die vorliegende Offenbarung gemäß einem zweiten Aspekt ein System zur Elektrostimulation des Auges mit einer Vorrichtung nach einem der vorhergehenden Aspekte und einer Stimulationselektrodenanordnung zur Elektrostimulation des Auges, wobei das in Eingriff mit einem Auge des Nutzers zu bringende Element die eine Stimulationselektrode ist und wobei der Halter ein Elektrodenhalter für die Stimulationselektrode ist. Der Halter und die Vorrichtung können ausgestaltet sein wie vorstehend beschrieben oder im Rahmen der vorliegenden Offenbarung beispielhaft erläutert.

Der Halter kann die Stimulationselektrode direkt oder indirekt halten. Die Stimulationselektrodenanordnung weist die Stimulationselektrode auf. Die Stimulationselektrodenanordnung kann jedoch auch beispielswiese einen oder mehrere Elektrodenträger aufweisen und über die Elektrodenträger am Halter befestigt sein.

Das Nasenteil kann dabei einen Nasenbügel und zwei Augenbügel aufweisen, an denen jeweils ein Elektrodenhalter befestigt wird.

Die Augenbügel sind dabei weiter vorzugsweise gegeneinander elektrisch isoliert und einzeln kontaktiert, so dass eine Therapie mit unterschiedlichen Stromstärken für die beiden Augen möglich ist.

Die Anordnung zum Halten des Gestells weist dabei in an sich bekannter Weise zwei an dem Nasenteil befestigte, vorzugsweise klappbar befestigte Seitenbügel auf. Die Anordnung zum Halten des Gestells kann auch breitenvariable, insbesondere federnd breitenvariable Seitenbügel aufweisen.

Das Gestell kann einen Stirnanschlag aufweisen. Im aufgesetzten Zustand kann der Stirnanschlag an einer Stirn des Nutzers anliegen und eine Bewegung der Vorrichtung in Richtung der Augen des Nutzers begrenzen. Dies kann gegenüber einer Vorrichtung, welche nur ein Nasenteil aufweist und auf dem Rasenrücken vor und zurück verschobene werden kann vorteilhaft sein. Insbesondere kann mit dem Stirnanschlag die Positioniergenauigkeit auch in der Eingriffsposition weiter verbessert werden und vorzugweise vermieden werden, dass in der Eingriffsposition ein zu hoher Druck durch das in Eingriff mit dem Auge des Nutzers zu bringende Element auf das Auge des Nutzers wirkt.

Allgemein ist es bei einer Vorrichtung und einem System zur Elektrostimulation noch bevorzugt, wenn eine Gegenelektrode für die Stimulationselektrode vorgesehen ist.

Ferner betrifft die vorliegende Offenbarung gemäß einem weiteren Aspekt ein Verfahren, insbesondere Verfahren zum Applizieren einer Stimulationselektrode einer Vorrichtung zur Elektrostimulation des Auges auf dem Auge des Nutzers, wobei das Verfahren folgende Schritte aufweist:
a) Bereitstellen einer Vorrichtung, insbesondere zur Elektrostimulation des Auges, wobei die Vorrichtung ein brillenartiges Gestell mit einem Nasenteil, eine mit dem Nasenteil verbundene Anordnung zum Halten des Gestells an dem Kopf des Nutzers sowie zumindest einen an dem Gestell angeordneten Halter für ein in Ein-griff mit einem Auge des Nutzers zu bringendes Element, insbesondere für eine drahtförmige Stimulationselektrode, aufweist, wobei der Halter dazu eingerichtet ist, zwischen einer Nicht-Eingriffsposition und einer vordefinierten Eingriffsposition hin und her bewegt zu werden, insbesondere wie im Rahmen der vorliegenden Offenbarung beschrieben;
b) Aufsetzen der Vorrichtung auf den Kopf des Nutzers, wobei sich der Halter in der Nicht-Eingriffsposition befindet; und
c) Bewegen des Halters von der Nicht-Eingriffsposition in die vordefinierte Eingriffsposition nachdem die Vorrichtung auf dem Kopf des Nutzers aufgesetzt ist, um das in Eingriff mit dem Auge des Nutzers zu bringende Element in Eingriff mit dem Auge des Nutzers zu bringen.

Die für die Vorrichtung gemäß dem ersten Aspekt beschriebenen weiteren optionalen Ausgestaltungen und Weiterbildungen gelten entsprechend für die weiteren Aspekte der vorliegenden Offenbarung, also insbesondere für das System, insbesondere zur Elektrostimulation des Auges, und das Verfahren.

Weitere Vorteile ergeben sich aus der Beschreibung der beigefügten Zeichnung.

Ausführungsbeispiele von Aspekten der Erfindung sind in den Zeichnungen dargestellt und werden in der nachfolgenden Beschreibung näher erläutert. Es zeigen:
- Fig. 1: eine schematische perspektivische einer Vorrichtung, insbesondere zur Elektrostimulation des Auges, mit einem Halter in der Nicht-Eingriffsposition;
- Fig. 2: eine schematische Seitenansicht der Vorrichtung mit dem Halter in der Nicht-Eingriffsposition;
- Fig. 3: eine schematische Seitenansicht der Vorrichtung mit dem Halter in der vordefinierten Eingriffsposition;
- Fig. 4: eine schematische perspektivische Darstellung eines brillenartigen Gestells;
- Fig. 5: eine schematische Seitenansicht eines Halters in einem ersten Zustand;
- Fig. 6: eine schematische Seitenansicht des Halters in einem zweiten Zustand;
- Fig. 7: eine schematische perspektivische Darstellung des Halters mit einer Elektrodenanordnung zur Elektrostimulation des Auges;
- Fig. 8: ein Flussdiagramm eines Verfahrens zum Befestigen einer Stimulationselektrodenanordnung an einer Vorrichtung zur Elektrostimulation des Auges
- Fig. 9: eine schematische Ansicht eines Ausschnitts der Vorrichtung mit dem Halter mit einer Elektrodenanordnung;
- Fig. 10: eine schematische Ansicht eines Betätigungsgriffs mit einer korrespondierenden Rasteinrichtung;
- Fig. 11: eine weitere schematische perspektivische Ansicht eines Ausschnitts der Vorrichtung mit dem Halter mit einer Elektrodenanordnung;
- Fig. 12: eine schematische Schnittdarstellung mit dem Betätigungsgriff und der korrespondierenden Rasteinrichtung aus Fig. 10;
- Fig. 13: eine weitere schematische Schnittdarstellung eines Ausschnitts der Vorrichtung.

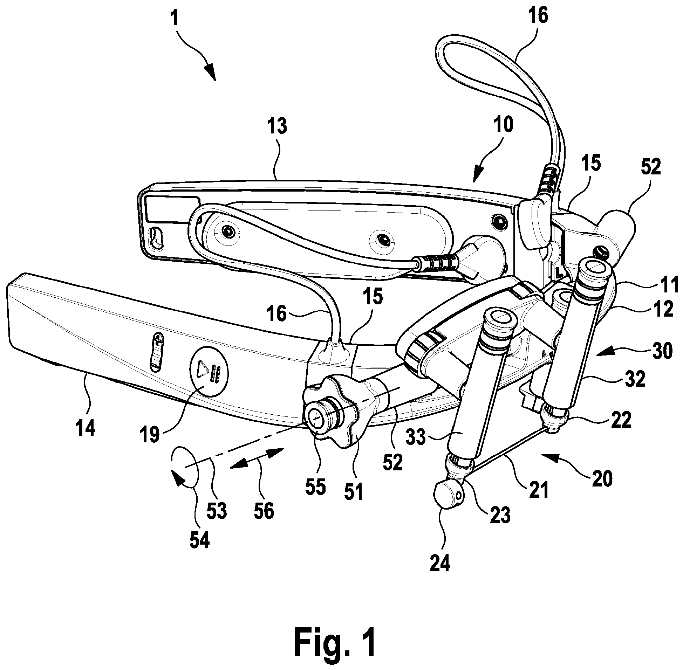

In Fig. 1 ist als Beispiel zur Veranschaulichung ein System 1 gezeigt, das zur Elektrostimulation des Auges eines Nutzers bzw. Patienten dient. Das System 1 weist eine Vorrichtung 10 mit einem Halter 30 und einem in Eingriff mit einem Auge des Nutzers zu bringendem Element 20 auf. Im vorliegenden Beispiel handelt es sich um eine Vorrichtung zur Elektrostimulation des Auges, bei welcher eine Stimulationselektrodenanordnung 20 in Kontakt mit dem Auge gebracht werden soll.

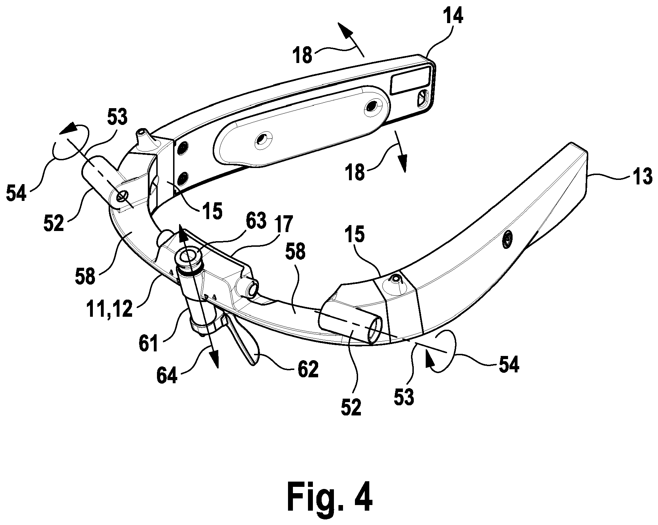

Die Vorrichtung 10 umfasst ein brillenartiges Gestell 11, mit einem Nasenteil 12 und einer mit dem Nasenteil 12 verbundene Anordnung zum Halten des Gestells am Kopf des Patienten. Die Anordnung zum Halten des Gestells 11 am Kopf des Patienten kann zwei Seitenbügel 13, 14 aufweisen, wie bei einer Brille üblich. Optional können die Seitenbügel 13, 14 klappbar an dem Nasenteil 12 angelegt werden. Die Seitenbügel 13, 14 können auch über Federelemente 15 breitenvariabel mit dem Nasenteil 12 verbunden sein. Auf diese Weise ist es möglich, die Vorrichtung flexibel an den Kopf des Nutzers anzupassen, wie mit den Pfeilen 18 in Fig. 4dargestellt.

Die Vorrichtung bzw. das Gestell 11 kann optional einen Stirnanschlag 17 aufweisen, wie in Fig. 4 gezeigt. Im aufgesetzten Zustand kann der Stirnanschlag an einer Stirn des Nutzers anliegen und eine Bewegung der Vorrichtung in Richtung der Augen des Nutzers begrenzen. Dies kann gegenüber einer Vorrichtung ohne Stirnanschlag, welche beispielsweise nur ein Nasenteil aufweist und auf dem Rasenrücken vor und zurück verschobene werden kann, vorteilhaft sein. Beispielsweise kann damit vermieden werden, dass das in Eingriff mit dem Auge des Nutzers zu bringende Element einen zu großen Druck auf das Auge ausübt.

Die Vorrichtung weist ferner einen an dem Gestell 11 angeordneten Halter 30 für ein in Eingriff mit einem Auge des Nutzers zu bringendes Element auf. Der Halter 30 ist dazu eingerichtet, zwischen einer Nicht-Eingriffsposition und einer vordefinierten Eingriffsposition hin und her bewegt zu werden.

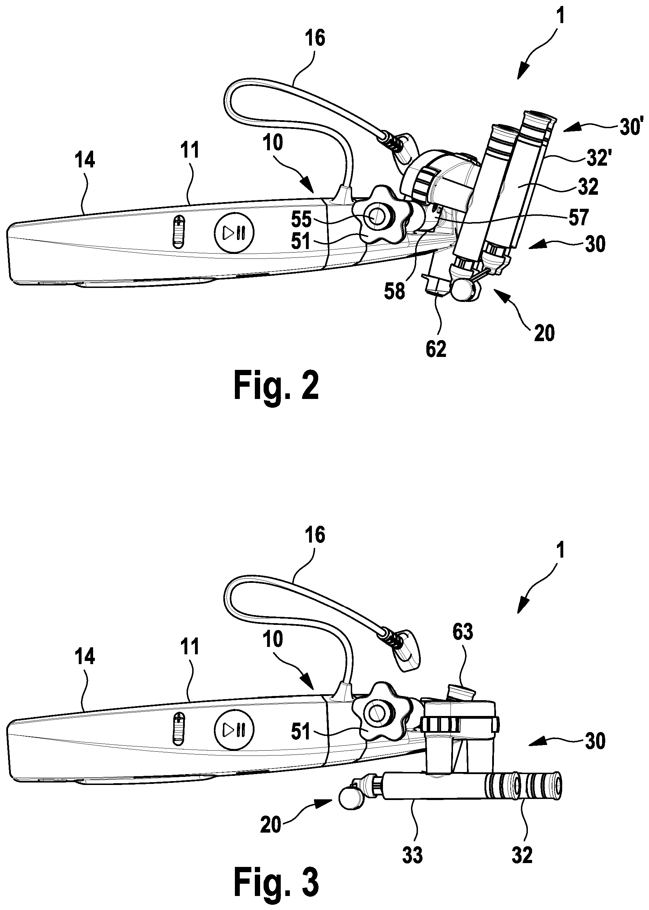

Fig. 1 und Fig. 2 zeigen den Halter 20 in der Nicht-Eingriffsposition. In Fig. 3 ist der Halter 20 in der vordefinierten Eingriffsposition gezeigt. In Fig. 4 ist das Gestell 11 ohne Halter dargestellt.

Im vorliegenden Beispiel handelt es sich bei dem 30 Halter um einen Elektrodenhalter für eine Stimulationselektrodenanordnung 20 mit einer Stimulationselektrode 21. Der Halter 30 kann hierbei dazu eingerichtet zwischen der Nicht-Eingriffsposition und der vordefinierten Eingriffsposition hin und her geschwenkt zu werden. Insbesondere kann der schwenkbare Halter 30 zwischen einer angehobenen Position, als Nicht-Eingriffsposition, und einer definierten Behandlungsposition, als Eingriffsposition, hin und her bewegt werden. In dem in Fig. 1 gezeigten Beispiel befindet sich der Halter in einer angehobenen Position. Dies ermöglicht es einem Patienten, das brillenartige Gestell 11 mit dem Halter 30 in der Nicht-Eingriffsposition auf den Kopf aufzusetzen. Hierdurch wird die Handhabung erleichtert. In einem ersten Schritt wird lediglich das brillenartige Gestell aufgesetzt, es ist jedoch noch nicht erforderlich, dass auch die Stimulationselektrode 21 im gleichen Schritt auf das Auge aufgelegt wird.

Erst nach dem Aufsetzen des brillenartigen Gestells 11 wird der Halter 30 abgesenkt und in die vordefinierte Eingriffsposition gebracht. Für den Fall einer Vorrichtung zur Elektrostimulation des Auges kann die Stimulationselektrode 21 in der Eingriffsposition in Anlage mit der vorderen Oberfläche des Auges gelangen. Die Stimulationselektrode 21 wölbt sich dabei vorzugsweise so aus, dass sie sich passend an die Wölbung der Augenoberfläche, insbesondere der Cornea anlegen kann. Dies ist möglich, weil die Stimulationselektrode 21 in sich flexibel und über den Gewichtskörper 24 nachgiebig in dem zweiten Elektrodenträger 23 gehalten sein kann.

Die Vorrichtung 10 weist vorzugsweise zwei separate Halter 30, 30' auf. Zur Vereinfachung ist in Fig. 1 und Fig. 3 jeweils nur ein Halter dargestellt. Der zweite Halter 30' ist in Fig. 2 schematisch hinter dem ersten Halter 30 dargestellt. Die Vorrichtung 30 kann einen linken Halter 30' für ein in Eingriff mit einem linken Auge des Nutzers zu bringendes Element, insbesondere für eine Stimulationselektrode 21 für das linke Auge, und einen rechten Halter 30 für ein in Eingriff mit einem rechten Auge des Nutzers zu bringendes Element, insbesondere für eine Stimulationselektrode 21 für das rechte Auge aufweisen. Der linke Halter 30' und der rechte Halter 30 sind dazu eingerichtet, separat voneinander zwischen einer jeweiligen Nicht-Eingriffsposition und einer jeweiligen Eingriffsposition hin und her bewegt zu werden. Die für den rechten Halter 30 beschriebenen Merkmale gelten für den linken Halter 30' entsprechend.

Die Anordnung der separaten Halter für das rechte und linke Auge ergeben sich auch aus der perspektivischen Ansicht des brillenartigen Gestells 11 aus Fig. 4 mit den jeweiligen Aufnahmen 52 in welchen Drehachsen für die separaten Halter für das rechte und linke Auge gelagert werden können.

In dem in Fig. 1 gezeigten Beispiel kann der Halter 30 beispielsweise über einen seitlich angeordneten Betätigungsgriff 51 zwischen der angehobenen Nicht-Eingriffsposition (siehe Fig. 2) und der definierten Eingriffsposition (siehe Fig. 3) hin und her bewegt werden. Der Betätigungsgriff 51 ragt in horizontaler Richtung über das Gestell 11 hinaus.

Hierbei ist der Halter 30 mittels der Aufnahmen 52 drehbar bzw. schwenkbar am brillenartigen Gestell 11 gelagert. Beispielsweise mit einem Lager in der Aufnahme 52, wie für einen nicht abgebildeten Elektrodenhalter für ein linkes Auge gezeigt. Die Drehachse ist mit 53 bezeichnet. Die Drehung ist mit Bezugszeichen 54 bezeichnet. Die horizontale Achse 53 liegt im vorliegenden Beispiel in vertikaler Richtung oberhalb der Augen des Nutzers und stört den Blick des Nutzers damit nicht. Alternativ kann die Drehachse für die Drehbewegung eine vertikale Achse sein.

Bei der in Fig. 1 gezeigten Ausführungsform liegt die horizontale Drehachse 53 in der Eingriffsposition ferner in Blickrichtung weiter entfernt vom Auge des Nutzers als die Stimulationselektrode 21. Dies hat zur Folge, dass die Einschwenkbewegung in das Auge im Wesentlichen waagrecht, jedoch mit einem geringfügigen Winkel von schräg unten erfolgt, um die Stimulationselektrode 21 in Eingriff mit dem Auge des Nutzers zu bringen. Dies hat sich als vorteilhaft erwiesen, da das Unterlied vom Nutzer beim Einschwenken leicht nach unten gezogen werden kann. Die Wimpern sind damit nicht im Weg und das In-Eingriff-Bringen wird erleichtert.

Es sind jedoch auch andere Konfigurationen denkbar. Bevorzugt ist der Halter dazu eingerichtet, das in Eingriff mit dem Auge des Nutzers zu bringende Element in einem Winkel im Bereich +/- 30°, insbesondere in einem Winkel im Bereich +/-20° zur Waagrechten von vorne auf das Auge des Nutzers in die vordefinierte Eingriffsposition zu bringen.

Für das rechte Auge und das linke Auge können separate Halter 30, 30' vorgesehen sein. Zur Vereinfachung ist in Fig. 1 nur ein Halter 30, hier ein Elektrodenhalter, dargestellt.

An dem Elektrodenhalter 30 ist die Stimulationselektrodenanordnung 20 befestigt, vorzugsweise magnetisch. In dem gezeigten Beispiel weist die Stimulationselektrodenanordnung 20 einen ersten Elektrodenträger 22 und einen zweiten Elektrodenträger 23 auf. Die Stimulationselektrode 21 ist zwischen dem ersten Elektrodenträger 22 und dem zweiten Elektrodenträger 23 angeordnet. Der erste und/oder zweite Elektrodenträger sind dazu eingerichtet, magnetisch an dem Elektrodenhalter 30 befestigt zu werden.

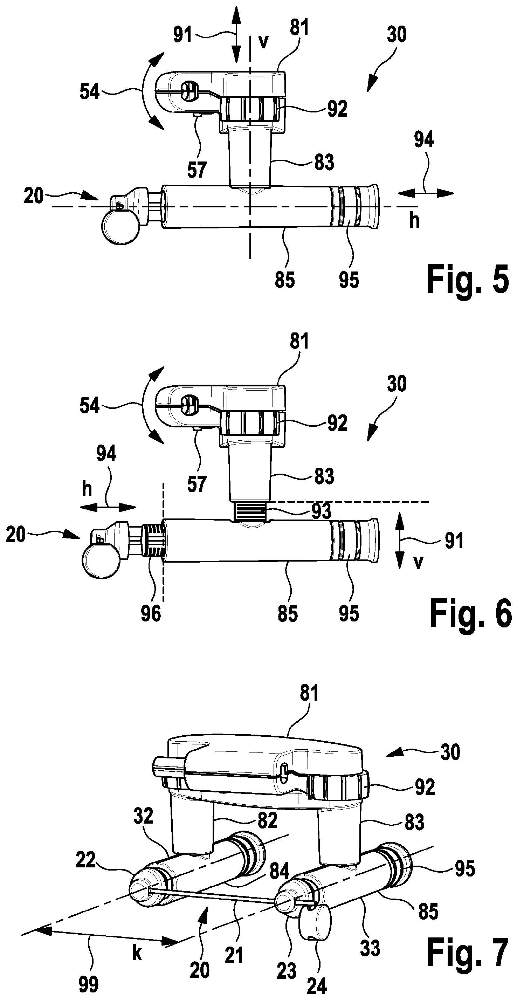

In dem in Fig. 1 gezeigten Beispiel weist der Elektrodenhalter 30 einen ersten, nasalen Elektrodenhalterteil 32 und einen zweiten, temporalen Elektrodenhalterteil 33 auf. Der erste, nasale Elektrodenhalterteil 32 kann auch als nasenseitiger Elektrodenhalterteil bezeichnet werden. Der zweite, temporale Elektrodenhalterteil 33 kann auch als schläfenseitiger Elektrodenhalterteil bezeichnet werden. Der erste Elektrodenträger 22 ist ein nasaler Elektrodenträger und dazu eingerichtet, magnetisch an einer Aufnahme des nasalen Elektrodenhalterteils 32 befestigt zu werden. Der zweite Elektrodenträger 23 ist ein temporaler Elektrodenträger und dazu eingerichtet, magnetisch an einer Aufnahme des temporalen Elektrodenhalterteils 33 befestigt zu werden. Der Aufbaut eines beispielhaften Halters 30 wird weiter unten in Bezug auf Fig. 5 bis 8 genauer erläutert.

Die Stimulationselektrode 21 ist im montierten Zustand elektrisch leitend mit einem Stimulationsgerät zur Elektrostimulation des Auges verbunden. Vorzugsweise erfolgt die elektrische Verbindung über den Elektrodenhalter 30. Das Stimulationsgerät erzeugt die gewünschten elektrischen Signale zur Elektrostimulation. Als Stimulationsgerät kann jedes beliebige Stimulationsgerät verwendet werden, das geeignete Stimulationssignale, insbesondere Sinuswellen oder pulsförmige elektrische Stimulationssignale für die Behandlung des Auges abgeben kann.

Das Stimulationsgerät kann in das brillenartige Gestell 11 integriert sein. Dies ist in Fig. 1 schematisch durch Bezugszeichen 19 und Bedienelemente zum Starten bzw. Pausieren der Elektrostimulation dargestellt. Die Stimulationseinrichtung kann jedoch auch als externe Stimulationseinrichtung vorgesehen sein.

Um den Stromkreis für die Elektrostimulation zu schließen können ferner Gegenelektroden 16 vorgesehen sein, welche beispielsweise an einer Stirn oder an einem Ohrläppchen des Patienten angebracht werden können.

Im Rahmen der vorliegenden Offenbarung kann es sich bei dem Stimulationsgerät um ein Gerät zum Stimulieren und/oder Messen handeln. Beispielsweise kann ein Stimulationssignal erzeugt werden, welches über die Stimulationselektrodenanordnung 20 mit der Stimulationselektrode 21 appliziert werden kann. Es kann sich jedoch auch beispielsweise um ein ERG-Gerät handeln, mit welchem ein Elektroretinogramm aufgezeichnet werden kann, welches mit der Stimulationselektrode 21 der Stimulationselektrodenordnung 20 erfasst wird.

Zwischen der in Fig. 2 gezeigten Nicht-Eingriffsposition und der in Fig. 3 gezeigten vordefinierten Eingriffsposition können ein oder mehrere vordefinierte Zwischenpositionen (nicht dargestellt) vorgesehen sein. Der Halter 30 kann dazu eingerichtet sein, über mindestens eine vordefinierte Zwischenposition, welche sich zwischen der Nicht-Eingriffsposition und der Eingriffsposition befindet, zwischen der Nicht-Eingriffsposition und der Eingriffsposition hin und her bewegt zu werden, insbesondere wobei eine Rasteinrichtung vorgesehen ist, um den Halter 30 in der vordefinierten Zwischenposition zu halten.

Zur Anpassung der Positionierung des Halters 30 gegenüber dem Gestell 11 kann der Halter 30 dazu eingerichtet sein in horizontaler Richtung 56, d.h. in Längsrichtung der Achse 53 verschoben zu werden. Dies ermöglicht eine Anpassung der Vorrichtung 10 für unterschiedliche Augenabstände. Ein Bedienelement 55 für diese horizontale Verschiebung, beispielsweise mittels einer im Halter 30 angeordneten Gewindespindel, kann in dem Betätigungsgriff 51 angeordnet sein, wie in Fig. 1 und Fig. 2 beispielhaft dargestellt.

Wie in Fig. 2 und Fig. 4 gezeigt kann an dem Gestell 11 ein Endanschlag 58 vorgesehen sein an welchen der Halter 30 in der vordefinierten Eingriffsposition anliegt. Hierfür kann der Halter 30 eine korrespondierende Anschlagfläche 57 aufweisen, wie in Fig 5 gezeigt. In der vordefinierten Eingriffsposition liegt die Anschlagfläche 57 des Halters 30 an dem Endanschlag 58 des Gestells 11 an, wie in Fig. 3 gezeigt. Entsprechend liegt die Anschlagfläche 57 in der Nicht-Eingriffsposition nicht an dem Endanschlag 58 an.

Alternativ kann die vordefinierte Eingriffsposition auch anderweitig begrenzt bzw. definiert werden, beispielsweise indem die Drehung 54 um die Rotationsachse 53 begrenzt wird. Dies kann beispielsweise auch mit einem Endanschlag in den jeweiligen Aufnahmen 52 erreicht werden.

In der Seitenansicht in Fig. 2 ist ferner eine Nasenstütze 62 gezeigt, mit welcher das Gestell 11 der Vorrichtung 10 auf einer Nase des Nutzers abgestützt werden kann. Die Nasenstütze kann ebenfalls anpassbar sein. Beispielsweise kann die Nasenstütze 62 längen- bzw. höhenverstellbar 64 sein mit einem Bedienelement 63, wie in Fig. 3 und Fig. 4 gezeigt.

Mit Bezug auf Fig. 5, Fig. 6 und Fig. 7 wird ein Ausführungsbeispiel eines Halters gemäß Aspekten der vorliegenden Offenbarung näher erläutert. Der Halter 30 ist dazu eingerichtet an einem Gestell 11, wie beispielsweise in Fig. 1 bis Fig. 4 gezeigt, angebracht zu werden, wobei die Vorrichtung 10 mit dem Gestell 11 und dem Halter 30 derart eingerichtet ist, zwischen einer Nicht-Eingriffsposition und einer vordefinierten Eingriffsposition hin und her bewegt zu werden. Beispielsweise mittels einer Schwenkbewegung wie mit dem Pfeil 54 schematisch dargestellt. Damit das in Eingriff mit dem Auge des Nutzers zu bringende Element korrekt platziert ist, kann der Halter 30 mehrere Einstellmöglichkeiten aufweisen.

In dem gezeigten Beispiel handelt es sich erneut um eine Vorrichtung zur Elektrostimulation des Auges mit einer Stimulationselektrodenanordnung 20 mit einem nasalen Elektrodenträger 22, einem temporalen Elektrodenträger 34, sowie einer dazwischen angeordneten Stimulationselektrode 21, an welche sich eine Gewichtskörper 24 anschließt, um die Stimulationselektrode 21 zu spannen. Der nasale Elektrodenträger 22 ist an einem verstellbaren nasalen Elektrodenhalterteil 32 befestigt. Der temporale Elektrodenträger 23 ist an einem verstellbaren temporalen Elektrodenhalterteil 33 befestigt. Ein Abstand 99 in horizontaler Richtung ist vorzugweise ein konstanter Abstand k. Die Stimulationselektrodenanordnung kann ebenfalls derart bereitgestellt werden, beispielweise in einer Blisterpackung, dass der nasale Elektrodenträger 22 und der temporale Elektrodenträger 23 mit dem gleichen konstanten Abstand k bereitgestellt werden und auf einfache Art und Weise anbringbar sind.

Wie In Fig. 5 und Fig. 6 gezeigt kann der Halter 30 eine Anschlagfläche 57, insbesondere eine anpassbare Anschlagfläche 57, aufweisen. Je nachdem wie weit die Anschlagfläche 57 herausragt, kann eine Neigung des Halters 30 gegenüber dem Gestell 11 mit dem Endanschlag 58 (siehe Fig. 4) variiert werden.

Wie in Fig. 5 bis Fig. 7 gezeigt, weist der Halter 30 einen horizontalen Quersteg 81 auf. Der Halter weist ferner einen nasalen vertikalen Schenkel 82 und einen temporalen vertikalen Schenkel 83 auf, welche sich von dem horizontalen Quersteg 81 des Halters 30, in vertikaler Richtung nach unten erstrecken. Der nasale vertikale Schenkel 82 und der temporale vertikale Schenkel 83 sind orthogonal zum Quersteg 81 des Halters 30 angeordnet. Im Rahmen der vorliegenden Offenbarung ist unter "orthogonal" eine im Wesentlichen orthogonale Anordnung zu versehen, insbesondere eine Anordnung in einem Winkel von 90° +/-30°, insbesondere +/-20°, insbesondere +/-10°. Der nasale vertikale Schenkel 82 und/oder der temporale vertikale Schenkel 83, vorzugweise beide, sind längenverstellbar. Hierdurch kann die Position des in Eingriff mit einem Auge des Nutzers zu bringenden Elements, hier der Stimulationselektrodenanordnung 20, angepasst werden. Dies ist in Fig. 6 durch den Pfeil 91 für eine Anpassung in vertikaler Richtung v gezeigt.

In der Seitenansicht Fig. 5 ist der temporale vertikale Schenkel 83 im eingefahrenen Zustand gezeigt. In der Seitenansicht in Fig. 6 ist der temporale vertikale Schenkel 83 im ausgefahrenen Zustand 93 gezeigt. Beispielsweise kann die Anpassung mittels einem Drehrad 92 und einer (innenliegenden) Gewindespindel erfolgen. Es können mehrere Markierungen vorgesehen sein, um eine definierte Längenverstellung zu erleichtern. Ein Vorteil der vorgeschlagenen Lösung kann darin bestehen, dass eine werkzeuglose Einstellung ermöglicht wird. Insbesondere kann mit der vorgeschlagenen Lösung mit Gewindespindeln eine feine Einstellung vorgenommen werden und gleichzeitig eine einfache Bedienbarkeit für sehbehinderte Nutzer ermöglicht wird. Die Längen der Schenkel 83 können beispielsweise mittels Gewindespindeln werkzeuglos verändert und optional nach manueller Einstellung fixiert werden.

Wie in Fig. 5 bis Fig. 7 gezeigt, kann der Halter 30 ferner einen nasalen horizontalen Schenkel 84 und einen temporalen horizontalen Schenkel 85 aufweisen. Der nasale horizontale Schenkel 84 ist an einem unteren (längenverstellbaren) Ende des nasalen vertikalen Schenkels 82 angeordnet. Der temporale horizontale Schenkel 85 ist an einem unteren (längenverstellbaren) Ende des temporalen vertikalen Schenkels 83 angeordnet. Die vertikalen und horizontalen Schenkel können orthogonal zueinander angeordnet sein. Der nasale horizontale Schenkel 84 weist eine erste Aufnahme für das in Eingriff mit dem Auge des Nutzers zu bringende Element auf, hier für den ersten Elektrodenträger 22 der Stimulationselektrodenanordnung 20. Der temporale horizontale Schenkel 85 weist eine zweite Aufnahme für das in Eingriff mit dem Auge des Nutzers zu bringende Element auf, hier für den zweiten Elektrodenträger 23 der Stimulationselektrodenanordnung 20. Der nasale horizontale Schenkel 84 und/oder der temporale horizontale Schenkel 85, vorzugsweise beide, sind längenverstellbar sind. Hierdurch kann die Position des in Eingriff mit einem Auge des Nutzers zu bringenden Elements, hier der Stimulationselektrodenanordnung 20, angepasst werden. Dies ist in Fig. 6 durch den Pfeil 94 für eine Anpassung in horizontaler Richtung h gezeigt.

In der Seitenansicht Fig. 5 ist der temporale horizontale Schenkel 85 im eingefahrenen Zustand gezeigt. In der Seitenansicht in Fig. 6 ist der temporale horizontale Schenkel 85 im ausgefahrenen Zustand 96 gezeigt. Beispielsweise kann die Anpassung mittels einem Drehrad 95 und einer (innenliegenden) Gewindespindel erfolgen. Es können mehrere Markierungen vorgesehen sein, um eine definierte Längenverstellung zu erleichtern.

Wie in Fig. 7 gezeigt kann zwischen den Schenkeln 82, 83 des Halters ein freier optischer Pfad in Blickrichtung des Nutzers bereitgestellt werden. Dies erhöht insbesondere bei längeren Anwendungen den Tragekomfort und die Akzeptanz beim Nutzer.

Sobald der Elektrodenhalter 30 angepasst ist, kann der Elektrodenhalter mit der fixiert angepassten Konfiguration zwischen der Nicht-Eingriffsposition und der definierten Eingriffsposition hin und her bewegt werden.

Es versteht sich, dass der Halter auch für ein anderes in Eingriff mit einem Auge des Nutzers zu bringendes Element, wie beispielsweise eine Kontaktlinse, eingerichtet sein kann. In diesem Fall kann es ausreichen nur einen mittig an Quersteg 81 angeordneten vertikalen Schenkel 83 und horizontalen Schenkel 85 vorzusehen mit einer Aufnahme beispielsweise für eine Kontaktlinse.

Der Halter selbst muss das Auge des Nutzers nicht notwendigerweise direkt kontaktieren. Es können auch ein oder mehrere Zwischenelemente vorgesehen sein, wie im vorliegenden Beispiel durch die Stimulationselektrodenanordnung gezeigt. Ferner kann ein Eingriff mit dem Auge des Nutzers nicht nur durch Kontaktierung der Cornea erfolgen, sondern auch durch Kontaktierung von einer oder mehrerer Regionen, insbesondere Hautbereichen, im Bereich des Auges. Auch eine transkutane Messung und/oder Stimulation ist denkbar.

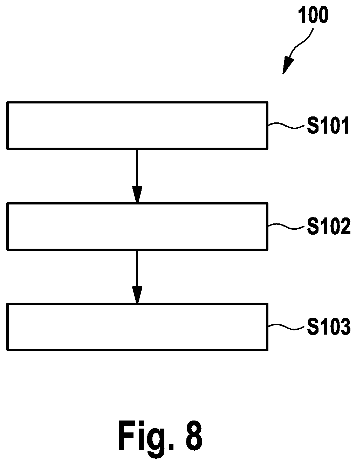

Fig. 8 zeigt ein Flussdiagramm eines Verfahrens 100, insbesondere ein Verfahren zum Applizieren einer Stimulationselektrode einer Vorrichtung zur Elektrostimulation des Auges auf dem Auge des Nutzers.

In einem ersten Schritt S101 wird eine Vorrichtung wie hierein offenbart bereitgestellt.

In einem zweiten Schritt S102 wird die Vorrichtung auf den Kopf des Nutzers aufgesetzt, wobei sich der Halter in der Nicht-Eingriffsposition befindet.

In einem dritten Schritt S103, nachdem die Vorrichtung auf dem Kopf des Nutzers aufgesetzt ist, wird der Halter von der Nicht-Eingriffsposition in die vordefinierte Eingriffsposition bewegt, um das in Eingriff mit dem Auge des Nutzers zu bringende Element in Eingriff mit dem Auge des Nutzers zu bringen.

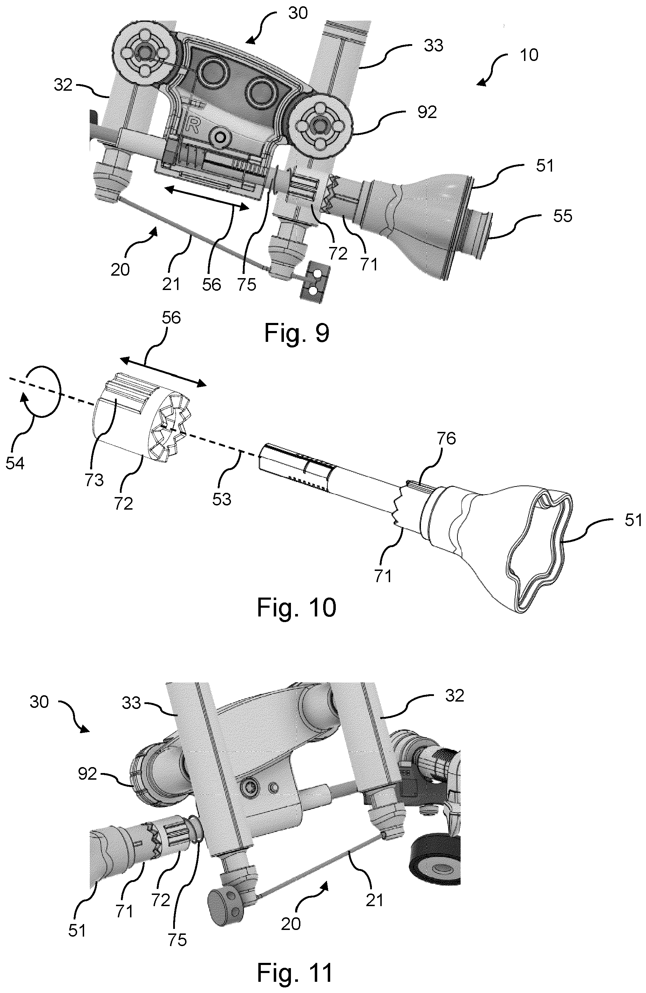

Weitere mögliche Ausgestaltungen der vorgeschlagenen Vorrichtung 10 werden nachfolgend mit Bezugnahme auf Fig. 9 bis 13 erläutert. Elemente, welche vorstehend bereits eingeführt wurden, sind mit den gleichen Bezugszeichen bezeichnet. Nachfolgend sollen optionale vorteilhafte Weiterbildungen und/oder Unterschiede hervorgehoben werden.

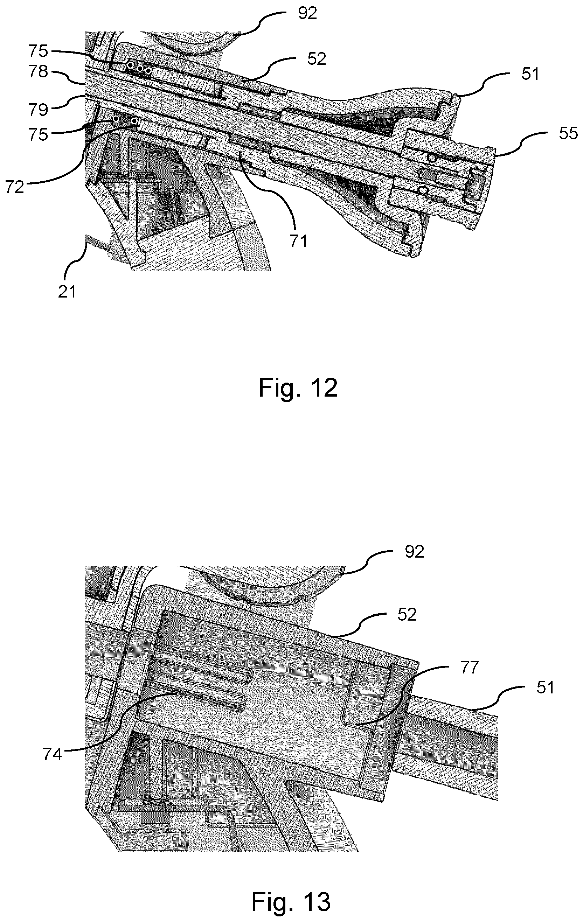

Fig. 9 zeigt eine schematische Teilschnittdarstellung eines Ausschnitts von Bestandteilen der Vorrichtung 10 mit dem Halter 30 mit einer Elektrodenanordnung 20 in einer Ansicht von einer Oberseite. Hierbei ist eine Schnittdarstellung durch den Halter 30 gezeigt. Der Halter 30 ist mittels der Aufnahmen 52 drehbar bzw. schwenkbar am brillenartigen Gestell 11 gelagert (siehe Fig. 4). Die Aufnahme 52 ist in Fig. 9 und Fig. 10 jedoch nicht abgebildet, um die innenliegenden Elemente darzustellen. In den Darstellungen Fig. 12 und Fig. 13 ist die Aufnahme 52 jeweils mit abgebildet.

In dem gezeigten Bespiel ist in der Aufnahme 52 eine Rasteinrichtung vorgesehen. Die Rasteinrichtung kann dazu eingerichtet sein, den Halter 30 in der vordefinierten Eingriffsposition aber auch in einer Nicht-Eingriffsposition zu halten. Wie vorstehend bereits beschreiben können zwischen der in Fig. 2 gezeigten Nicht-Eingriffsposition und der in Fig. 3 gezeigten vordefinierten Eingriffsposition ein oder mehrere vordefinierte Zwischenpositionen vorgesehen sein. Der Halter 30 kann dazu eingerichtet sein, über mindestens eine vordefinierte Zwischenposition, welche sich zwischen der Nicht-Eingriffsposition und der Eingriffsposition befindet, zwischen der Nicht-Eingriffsposition und der Eingriffsposition hin und her bewegt zu werden. Die Rasteinrichtung ist im vorliegenden Beispiel dazu eingerichtet, um den Halter 30 in mehreren vordefinierten Zwischenpositionen zu halten. Die Rasteinrichtung kann hierbei dazu eingerichtet sein, in verschiedenen Einrastpositionen jeweils eine lösbare Formschlussverbindung bereitzustellen, um den Elektrodenhalter 30 gegenüber dem Gestell 11 in der vordefinierten Eingriffsposition, in der Nicht-Eingriffsposition und optional in einer oder mehreren Zwischenpositionen zu halten.

Beispielsweise kann die Rasteinrichtung durch korrespondierende Rastelemente 71, 72 bereitgestellt werden. Ein erstes Rastelement 71 ist am Halter 30 oder am Betätigungsgriff angeordnet. Ein zweites Rastelement 72 ist am Gestell 11 angeordnet, insbesondere an der Aufnahme 52. Die Rastelemente können beispielsweise als korrespondierende Zahnkränze ausgebildet sein, welche in mehreren definierten Winkelpositionen ineinandergreifen können. Die Rastelemente sind also dazu ausgebildet, definierte Formschlussverbindungen in verschiedenen (vorgegebenen) Winkelpositionen bereitzustellen. In einer Ausgestaltung unterscheiden sich eine erste und eine zweite Winkelposition um zumindest 5°, insbesondere um zumindest 15°, insbesondere um zumindest 30°, insbesondere um zumindest 45°, insbesondere um zumindest 60°. Alternativ oder zusätzlich unterscheiden sich eine erste und eine zweite Winkelposition um höchsten 270°, insbesondere um höchstens 225°, insbesondere um höchstens 180°, insbesondere um höchstens 150°. Bei der dem Unterschied kann es sich um einen maximalen Winkel zwischen der definierten Eingriffsposition und einer Nicht-Eingriffsposition handeln. Somit kann ein begrenzter Winkelbereich für eine Einschwenkbewegung vorgesehen sein, wodurch die Handhabung erleichtert werden kann.

Im Rahmen der vorliegenden Offenbarung ist vorzugsweise keine Reibschlussverbindung vorgesehen. Bei einer Reibschlussverbindung muss eine der Reibungskraft entgegenwirkende Kraft aufgewendet werden, um den Reibschluss zu lösen. Diese Kraft bewirkt ein Drehmoment mit einer Drehachse, die bei einer vom Auge wegführenden Bewegung im Auflagepunkt des Seitenbügels auf dem Ohr liegt und bei einer zum Auge hinführenden Bewegung auf dem Auflagepunkt der Nasenstütze auf dem Nasenrücken. Soll die Bewegung vom Auge weg erfolgen, bewirkt dies, dass die gesamte Vorrichtung sich nach oben bewegt, mindestens solange bis der Reibschluss gelöst ist. Um eine Verdrehung und Verrutschen der auf der Nase gelagerten Vorrichtung zu verhindern, insbesondere wenn bereits eine der Elektroden in Kontakt mit einem Auge gebracht wurde, müsste mit der zweiten Hand eine der Rotationsrichtung entgegenwirkende Gegenkraft auf die Vorrichtung ausgeübt werden. Damit müssten zum Heranschwenken der Elektroden an das Auge immer beide Hände an der Vorrichtung sein. Das hätte den Nachteil, dass keine Hand frei ist, um die Augenlider nach unten bzw. nach oben zu schieben, sprich um das Auge beim Aufbringen der Stimulationselektrode offen zu halten.

Die vorgeschlagene Formschlussverbindung mittels einer Rasteinrichtung kann demgegenüber einen oder mehrere Vorteile bieten. Die Rasteinrichtung kann dazu eingerichtet sein, dass ein Formschluss durch eine im Lager 52 überwiegend parallel zur Achse 53 wirkende Kraft gelöst wird. Dies kann beispielsweise durch zwei insbesondere konzentrisch auf die Achse 53 aufgebrachte korrespondierende Rastelemente 71, 72 erreicht werden. Eine schematische Ansicht eines Betätigungsgriffs 51 mit einem ersten Rastelement 71 und einem korrespondierenden Rastelement 72 ist in Fig. 10 dargestellt.

Durch das Lösen des Formschlusses entsteht im Gegensatz zum Reibschluss keine bzw. allenfalls eine geringere Kraft, die zu einer Rotation um eine Drehachse im Auflagepunkt des Seitenflügels bzw. der Nasenstütze auf dem Nasenrücken führt. Für die Bewegung des Elektrodenhalters von einer Einrastposition in die nächste müssen, nachdem der Formschluss gelöst ist, keine Reibungskräfte mehr überwunden werden bzw. allenfalls geringere. Die Gefahr, dass durch die Drehbewegung eine bereits auf der Augenoberfläche positionierte Stimulationselektrode 21 verrutscht, wird damit reduziert oder vermieden werden.

Ein weiterer Vorteil kann darin bestehen, dass die Drehbewegung mit nur einer Hand ausgeführt werden kann. Die zweite Hand bleibt hingegen frei um z.B. das untere Augenlid nach unten zu ziehen, damit die Stimulationselektrode 21 der Stimulationselektrodenanordnung 20 auf der unteren Hälfte der Augenoberfläche positioniert werden kann, ohne beispielsweise beim Hereinschwenken die Wimpern zu berühren.

Das Rastelement 72 ist in dem geigten Ausführungsbeispiel vorzugweise drehfest mit dem Gestell bzw. mit der Aufnahme 52 verbunden. Allerdings kann das Rastelement in axialer Richtung entlang der Drehachse 54 verschieblich gelagert sein. Hierfür können das Rastelement 72 und die Aufnahme 52 korrespondierende Führungselemente 73, 74 aufweisen. Im vorliegenden Beispiel sind am Rastelement 72 Vertiefungen als Führungselement 73 vorgesehen, wie in Fig. 9, Fig. 10, und Fig. 11 dargestellt. An der Aufnahme 52 sind korrespondierende Stege als Führungselemente 74 vorgesehen, wie in Fig. 13 dargestellt. Hierbei kann in der Aufnahme 52 ein Federelement 75 vorgesehen sein, welches das Rastelement 72 in Richtung des korrespondierenden Rastelements 71 drückt. Das Federelement 75 definiert eine Kraft, welche zum Lösen des Formschlusses in axialer Richtung 56 überwunden werden muss.

Mit anderen Worten werden die Rastpositionen im vorliegenden Ausführungsbeispiel definiert durch die ineinandergreifenden Zacken des ersten Rastelements 71 und des zweiten Rastelements 72. Die Betätigung des Schwenkmechanismus über den Betätigungsgriff 51 bewirkt das gegeneinander verschieben der Zacken. Das Rastelement 72 kann in axialer Richtung bewegt werden. Das Rastelement 72 ist durch die beiden länglichen Nuten als Führungselement 74 im äußeren Teil, also in der Aufnahme 52 fixiert und kann nicht verdreht werden. Die Freiheit zur Drehung des korrespondierenden Rastelements 71 wird durch ein Federelement 75 realisiert. Durch dieses besteht die Flexibilität, um das Rastelement 72 zu drehen und eine Zacke weiter zu drehen. Vorzugseise kann das Führungselement sowohl auf einer Oberseite, wie in der Draufsicht in Fig. 9 gezeigt, als auch auf einer Unterseite, wie in der Ansicht in Fig. 11 gezeigt, vorgesehen sein.

Bezugnehmend auf die Darstellungen in Fig. 10 und Fig. 12, können der Halter 30 und das Gestell 11 dazu eingerichtet sein, eine Bewegung relativ zueinander mechanisch zu begrenzen. Insbesondere kann eine Begrenzung für einen Winkelbereich einer Bewegung relativ zueinander begrenzt sein. Der Halter 30 kann ein schwenkbarer Halter sein. Der Winkelbereich kann begrenz sein auf einen Winkelbereich von nicht mehr als 270°, insbesondere von nicht mehr als 180°, insbesondere von nicht mehr als 150°. In dem in Fig. 10 und Fig. 12 gezeigten Ausführungsbeispiel weise der Halter 30 bzw. der Betätigungsgriff 51 einen Anschlag 76 auf. Die Aufnahme 52 kann eine korrespondierenden Anschlag 77 bereitstellen. Beispielsweise kann der Anschlag 76 durch einen Steg oder einen erhabenen Abschnitt und der Anschlag 77 durch eine korrespondierende Ausnehmung bzw. Vertiefung oder Nut mit einer als Anschlag dienenden Seitenfläche bereitgestellt werden. Ein gewünschter Drehwinkelbereich kann damit auf einfache Art und Weise festgelegt werden. Es versteht sich, dass auch die Anschläge 76, 77 mehrfach, beispielsweise oben und unten bzw. um 180° versetzt bereitgestellt werden können. Die hier beschriebene Begrenzung kann alternativ oder zusätzlich zur Begrenzung durch die Anschlagflächen 57, 58, wie in Fig. 2 und Fig. 4 dargestellt, bereitgestellt werden, um eine vordefinierte Eingriffsposition bereitzustellen. Alternativ kann die vordefinierte Eingriffsposition also auch anderweitig begrenzt bzw. definiert werden, beispielsweise indem die Drehung 54 um die Rotationsachse 53 begrenzt wird. Dies kann beispielsweise auch mit einem Endanschlag in den jeweiligen Aufnahmen 52 erreicht werden.

Fig. 12 eine schematische Schnittdarstellung mit dem Betätigungsgriff 51 und der Rasteinrichtung mit den Rastelementen 71, 72. Optional kann der Halter 30 nicht nur gegenüber dem Gestell 11 verschwenkt werden. Zur Anpassung der Positionierung des Halters 30 gegenüber dem Gestell 11 kann der Halter 30 dazu eingerichtet sein in horizontaler Richtung 56, d.h. in Längsrichtung der Achse 53 verschoben zu werden. Dies ermöglicht eine Anpassung der Vorrichtung 10 für unterschiedliche Augenabstände. Die Verstellung in horizontaler Richtung ist in Fig. 1 und Fig. 9 mit Pfeil 56 dargestellt. Ein Bedienelement 55 für diese horizontale Verschiebung, beispielsweise mittels einer im Halter 30 angeordneten Gewindespindel, kann in dem Betätigungsgriff 51 angeordnet sein, wie in Fig. 1, Fig. 2, Fig. 9 und Fig. 12 beispielhaft dargestellt. Dies kann dadurch ermöglicht werden, dass zwei koaxiale Schäfte 78, 79 vorgesehen sind. Beispielsweise kann ein innerer Schaft 78 von einem äußeren Schaft 79 umgeben sein. Der innere Schaft 78 kann mit Bedienelement 78 gekoppelt sein. Eine Verschiebung kann beispielsweise mittels einer Gewindestange und einer Drehbewegung am Bedienelement 55 bereitgestellt werden. Ein Verschenken des Halters 30 relativ zum Gestell kann mittels einer Drehbewegung am Betätigungsgriff 51 bereitgestellt werden.

Zusammenfasend kann gemäß Aspekten der vorliegenden Offenbarung eine Handhabung einer Vorrichtung, insbesondere einer Vorrichtung zur Elektrostimulation des Auges, weiter verbessert und ein angenehmeres Nutzererlebnis ermöglicht werden. Insbesondere kann eine Applikation eines in Eingriff eines mit dem Auge des Nutzers zu bringenden Elements erleichtert werden.

## Patentansprüche

1. Vorrichtung (10), insbesondere Vorrichtung zur Elektrostimulation des Auges, wobei die Vorrichtung (10) ein brillenartiges Gestell (11) mit einem Nasenteil (12), eine mit dem Nasenteil (12) verbundene Anordnung zum Halten des Gestells an dem Kopf des Nutzers sowie zumindest einen an dem Gestell (11) angeordneten Halter (30) für ein in Eingriff mit einem Auge des Nutzers zu bringendes Element, insbesondere für eine drahtförmige Stimulationselektrode (21), aufweist, **dadurch gekennzeichnet, dass** der Halter (30) dazu eingerichtet ist, zwischen einer Nicht-Eingriffsposition und einer vordefinierten Eingriffsposition hin und her bewegt zu werden, wobei der Halter (30) einen nasalen vertikalen Schenkel (82) und einen temporalen vertikalen Schenkel (83) aufweist, welche sich von dem Halter, insbesondere von einem horizontalen Quersteg (81) des Halters, in vertikaler Richtung nach unten erstrecken, wobei der nasale vertikale Schenkel (82) und/oder der temporale vertikale Schenkel (83) längenverstellbar (92, 93) sind.

2. Vorrichtung (10), insbesondere Vorrichtung zur Elektrostimulation des Auges, wobei die Vorrichtung (10) ein brillenartiges Gestell (11) mit einem Nasenteil (12), eine mit dem Nasenteil (12) verbundene Anordnung zum Halten des Gestells an dem Kopf des Nutzers sowie zumindest einen an dem Gestell (11) angeordneten Halter (30) für ein in Eingriff mit einem Auge des Nutzers zu bringendes Element, insbesondere für eine drahtförmige Stimulationselektrode (21), aufweist, **dadurch gekennzeichnet, dass** der Halter (30) dazu eingerichtet ist, zwischen einer Nicht-Eingriffsposition und einer vordefinierten Eingriffsposition hin und her bewegt zu werden, wobei der Halter (30) einen nasalen horizontalen Schenkel (84) und einen temporalen horizontalen Schenkel (85) aufweist, wobei der nasale horizontale Schenkel (84) eine erste Aufnahme für das in Eingriff mit dem Auge des Nutzers zu bringende Element (20, 21) aufweist, wobei der temporale horizontale Schenkel (85) eine zweite Aufnahme für das in Eingriff mit dem Auge des Nutzers zu bringende Element (20, 21) aufweist, und wobei der nasale horizontale Schenkel (84) und/oder der temporale horizontale Schenkel (85) längenverstellbar (95, 96) sind.

3. Vorrichtung (10) nach Anspruch 1 oder 2, wobei die Vorrichtung eine Vorrichtung zur Elektrostimulation des Auges ist und wobei der Halter (30) ein Elektrodenhalter für eine Stimulationselektrodenanordnung (20) ist, insbesondere wobei die Stimulationselektrodenanordnung (20) einen ersten Elektrodenträger (22) und einen zweiten Elektrodenträger (23) aufweist, wobei die Stimulationselektrode (21) zwischen dem ersten Elektrodenträger (22) und dem zweiten Elektrodenträger (23) angeordnet ist, wobei der erste und/oder zweite Elektrodenträger dazu eingerichtet ist, magnetisch an dem Elektrodenhalter (30) befestigt zu werden.

4. Vorrichtung (10) nach einem der vorhergehenden Ansprüche, wobei die Vorrichtung zwei separate Halter aufweist, einen linken Halter (30') für ein in Eingriff mit einem linken Auge des Nutzers zu bringendes Element, insbesondere für eine Stimulationselektrode (21) für das linke Auge, und einen rechten Halter (30) für ein in Eingriff mit einem rechten Auge des Nutzers zu bringendes Element, insbesondere für eine Stimulationselektrode (21) für das rechte Auge; insbesondere wobei der linke Halter (30') und der rechte Halter (30) dazu eingerichtet sind, separat voneinander zwischen einer jeweiligen Nicht-Eingriffsposition und einer jeweiligen Eingriffsposition hin und her bewegt zu werden.

5. Vorrichtung (10) nach einem der vorhergehenden Ansprüche,
wobei der Halter (30) dazu eingerichtet ist, zwischen einer einstellbaren vordefinierten Nicht-Eingriffsposition und einer einstellbaren vordefinierten Eingriffsposition hin und her bewegt zu werden; und/oder
wobei in der vordefinierte Eingriffsposition eine Bewegung des Halters in Richtung des Auges des Nutzers mechanisch begrenzt ist, insbesondere wobei die Begrenzung einstellbar ist; und/oder
wobei der Halter (30) in der vordefinierten Eingriffsposition an einen von dem Gestell (11) bereitgestellten Endanschlag (58) anliegt.

6. Vorrichtung (10) nach einem der vorhergehenden Ansprüche, wobei der Halter (30) einen Betätigungsgriff (51) zur Bewegung des Halters zwischen der Nicht-Eingriffsposition und der Eingriffsposition aufweist, wobei
(a) der Betätigungsgriff (51) in horizontaler Richtung (56) über das Gestell (11) hinausragt; insbesondere wobei ein Bedienelement (55), um den Halter (30) in horizontaler Richtung (56) gegenüber dem Gestell (11) zu verstellen, in dem Betätigungsgriff (51) angeordnet ist; und/oder
(b) die Vorrichtung dazu eingerichtet ist, dass der Betätigungsgriff außerhalb eines Gesichtsfelds des Nutzers, insbesondere außerhalb eines zentralen Gesichtsfelds des Nutzers angeordnet ist; insbesondere wobei die Vorrichtung dazu eingereicht ist, dass der Betätigungsgriff (51) in der Eingriffsposition außerhalb eines zentralen Gesichtsfelds des Nutzers angeordnet ist.

7. Vorrichtung (10) nach einem der vorhergehenden Ansprüche, der Halter (30) dazu eingerichtet ist, einhändig und werkzeuglos, zwischen der Nicht-Eingriffsposition und der vordefinierten Eingriffsposition hin und her bewegt zu werden.

8. Vorrichtung (10) nach Anspruch 2 oder Anspruch 2 in Verbindung mit einem der vorhergehenden Ansprüche, wobei die erste Aufnahme des nasalen horizontalen Schenkels (84) und die zweite Aufnahme des temporalen horizontalen Schenkels (85) einen festen horizontalen Abstand (99) voneinander aufweisen.

9. Vorrichtung (10) nach Anspruch 1 und 2 oder Anspruch 1 und 2 in Verbindung mit einem der vorhergehenden Ansprüche, wobei sich der nasale vertikale Schenkel (82) und der temporale vertikale Schenkel (83) von dem horizontalen Quersteg (81) des Halters (30) in vertikaler Richtung nach unten erstrecken; wobei der nasale horizontale Schenkel (84) an einem unteren Ende des nasalen vertikalen Schenkels (82) angeordnet ist; und wobei der temporale horizontale Schenkel (85) an einem unteren Ende des temporalen vertikalen Schenkels (83) angeordnet ist; insbesondere wobei der nasale vertikale Schenkel (82) und der temporale vertikale Schenkel (83) orthogonal zum Quersteg (81) des Halters (30) angeordnet sind; wobei der nasale horizontale Schenkel (84) und der temporale horizontale Schenkel (85) orthogonal zum Quersteg (81) des Halters angeordnet sind; wobei der nasale horizontale Schenkel (84) orthogonal zum nasalen vertikalen Schenkel (82) angeordnet ist; und wobei der temporale horizontale Schenkel (85) orthogonal zum temporalen vertikalen Schenkel (83) angeordnet ist.

10. Vorrichtung (10) nach einem der vorhergehenden Ansprüche, wobei der nasale vertikale Schenkel (82) und/oder der temporale vertikale Schenkel (83) und/oder der nasale horizontale Schenkel (84) und/oder der temporale horizontale Schenkel (85) mit einer Gewindespindel längenverstellbar ist.

11. Vorrichtung (10) nach einem der vorhergehenden Ansprüche, wobei der Halter (30) dazu eingerichtet ist, das in Eingriff mit dem Auge des Nutzers zu bringende Element an einem ersten Auflagepunkt und einem zweiten Auflagepunkt in der Eingriffsposition in Eingriff mit dem Auge des Nutzers zu bringen, wobei der Halter (30) dazu eingerichtet ist, den ersten Auflagepunkt in drei Raumrichtungen zu justieren und den zweiten Auflagepunkt in drei Raumrichtungen zu justieren, insbesondere wobei genau eine Raumrichtung derart gekoppelt sind, das der erste und zweite Auflagepunkt einen definierten Abstand (99) in dieser Raumrichtung aufweisen.

12. Vorrichtung (10) nach einem der vorhergehenden Ansprüche, wobei in Eingriff mit dem Auge des Nutzers zu bringendes Element eine Kontaktlinse ist; wobei der Halter (30) ein Halter für die Kontaktlinse ist und dazu eingerichtet ist, die Kontaktlinse in der vordefinierten Eingriffsposition auf das Auge des Nutzers zu applizieren.

13. System (1) zur Elektrostimulation des Auges mit einer Vorrichtung (10) nach einem der vorhergehenden Ansprüche und einer Stimulationselektrodenanordnung (20) zur Elektrostimulation des Auges, wobei das in Eingriff mit einem Auge des Nutzers zu bringendes Element die eine Stimulationselektrode (21) ist und wobei der Halter (30) ein Elektrodenhalter für die Stimulationselektrode (21) ist.

14. Nichtchirurgisches Verfahren (100), insbesondere Verfahren zum Applizieren einer Stimulationselektrode einer Vorrichtung zur Elektrostimulation des Auges auf dem Auge des Nutzers, wobei das Verfahren folgende Schritte aufweist:
a) Bereitstellen (S101) einer Vorrichtung (10), insbesondere zur Elektrostimulation des Auges, wobei die Vorrichtung (10) ein brillenartiges Gestell (11) mit einem Nasenteil (12), eine mit dem Nasenteil (12) verbundene Anordnung zum Halten des Gestells an dem Kopf des Nutzers sowie zumindest einen an dem Gestell (11) angeordneten Halter (30) für ein in Eingriff mit einem Auge des Nutzers zu bringendes Element, insbesondere für eine drahtförmige Stimulationselektrode (21), aufweist, wobei der Halter (30) dazu eingerichtet ist, zwischen einer Nicht-Eingriffsposition und einer vordefinierten Eingriffsposition hin und her bewegt zu werden;
b) Aufsetzen (S102) der Vorrichtung auf den Kopf des Nutzers, wobei sich der Halter in der Nicht-Eingriffsposition befindet; und
c) Bewegen (S103) des Halters (30) von der Nicht-Eingriffsposition in die vordefinierte Eingriffsposition nachdem die Vorrichtung auf dem Kopf des Nutzers aufgesetzt ist, wobei in der vordefinierten Eingriffsposition eine weitere Bewegung des Halters in Richtung des Auges des Nutzers mechanisch begrenzt ist, insbesondere durch einen Anschlag, um das in Eingriff mit dem Auge des Nutzers zu bringende Element (21) in Eingriff mit dem Auge des Nutzers zu bringen.

## Claims

1. Device (10), in particular device for electrostimulation of the eye, wherein the device (10) comprises a spectacle-like frame (11) with a nose part (12), an arrangement connected to the nose part (12) for holding the frame on the head of the user, and at least one holder (30) arranged at the frame (11) for an element to be brought into engagement with an eye of the user, in particular for a wire-shaped stimulation electrode (21), **characterized in that** the holder (30) is adapted to be moved back and forth between a non-engagement position and a predefined engagement position, wherein the holder (30) comprises a nasal vertical leg (82) and a temporal vertical leg (83) which extend downward in a vertical direction from the holder, in particular from a horizontal crossbar (81) of the holder, wherein the nasal vertical leg (82) and/or the temporal vertical leg (83) are length-adjustable (92, 93).

2. Device (10), in particular a device for electrostimulation of the eye, wherein the device (10) comprises a spectacle-like frame (11) with a nose part (12), an arrangement connected to the nose part (12) for holding the frame on the head of the user, and at least one holder (30) arranged at the frame (11) for an element to be brought into engagement with an eye of the user, in particular for a wire-shaped stimulation electrode (21), **characterized in that** the holder (30) is adapted to be moved back and forth between a non-engagement position and a predefined engagement position, wherein the holder (30) comprises a nasal horizontal leg (84) and a temporal horizontal leg (85), wherein the nasal horizontal leg (84) comprises a first receptacle for the element (20, 21) to be brought into engagement with the eye of the user, and wherein the temporal horizontal leg (85) comprises a second receptacle for the element (20, 21) to be brought into engagement with the eye of the user, and wherein the nasal horizontal leg (84) and/or the temporal horizontal leg (85) are length-adjustable (95, 96).

3. Device (10) according to claim 1 or 2, wherein the device is a device for electrostimulation of the eye and wherein the holder (30) is an electrode holder for a stimulation electrode arrangement (20), in particular wherein the stimulation electrode arrangement (20) comprises a first electrode carrier (22) and a second electrode carrier (23), wherein the stimulation electrode (21) is arranged between the first electrode carrier (22) and the second electrode carrier (23), wherein the first and/or second electrode carrier is adapted to be magnetically attached to the electrode holder (30).

4. Device (10) according to any of the preceding claims, wherein the device comprises two separate holders, a left holder (30') for an element to be brought into engagement with a left eye of the user, in particular for a stimulation electrode (21) for the left eye, and a right holder (30) for an element to be brought into engagement with a right eye of the user, in particular for a stimulation electrode (21) for the right eye, in particular wherein the left holder (30') and the right holder (30) are adapted to be moved separately from each other between a respective non-engagement position and a respective engagement position.

5. Device (10) according to any of the preceding claims,
wherein the holder (30) is adapted to be moved back and forth between an adjustable predefined non-engagement position and an adjustable predefined engagement position; and/or
wherein, in the predefined engagement position, a movement of the holder toward the eye of the user is mechanically limited, in particular wherein the limitation is adjustable; and/or
wherein the holder (30) in the predefined engagement position abuts against an end stop (58) provided by the frame (11).

6. Device (10) according to any of the preceding claims, wherein the holder (30) has an actuating handle (51) for moving the holder between the non-engagement position and the engagement position, wherein
(a) the operating handle (51) projects in a horizontal direction (56) beyond the frame (11), in particular wherein an operating element (55) for adjusting the holder (30) in a horizontal direction (56) relative to the frame (11) is arranged in the operating handle (51); and/or
(b) the device is adapted such that the actuating handle is arranged outside a field of view of the user, in particular outside a central field of view of the user; in particular, the device is adapted such that the actuating handle (51) is arranged outside a central field of view of the user in the engagement position.

7. Device (10) according to any of the preceding claims, wherein the holder (30) is adapted to be moved back and forth between the non-engagement position and the predefined engagement position single-handedly and without tools.

8. Device (10) according to claim 2 or claim 2 in combination with any of the preceding claims, wherein the first receptacle of the nasal horizontal leg (84) and the second receptacle of the temporal horizontal leg (85) have a fixed horizontal distance (99) between each other.

9. Device (10) according to claims 1 and 2 or claims 1 and 2 in combination with any of the preceding claims, wherein the nasal vertical leg (82) and the temporal vertical leg (83) extend downward from the horizontal crossbar (81) of the holder (30) in a vertical direction; wherein the nasal horizontal leg (84) is arranged at a lower end of the nasal vertical leg (82); and wherein the temporal horizontal leg (85) is arranged at a lower end of the temporal vertical leg (83); in particular wherein the nasal vertical leg (82) and the temporal vertical leg (83) are arranged orthogonal to the crossbar (81) of the holder (30); wherein the nasal horizontal leg (84) and the temporal horizontal leg (85) are arranged orthogonal to the crossbar (81) of the holder; wherein the nasal horizontal leg (84) is arranged orthogonal to the nasal vertical leg (82); and wherein the temporal horizontal leg (85) is arranged orthogonal to the temporal vertical leg (83).

10. Device (10) according to any of the preceding claims, wherein the nasal vertical leg (82) and/or the temporal vertical leg (83) and/or the nasal horizontal leg (84) and/or the temporal horizontal leg (85) is length adjustable with a threaded spindle.

11. Device (10) according to any of the preceding claims, wherein the holder (30) is adapted to bring the element to be brought into engagement with the eye of the user at a first contact point and a second contact point into engagement with the eye of the user in the engagement position, wherein the holder (30) is adapted to adjust the first contact point in three spatial directions and to adjust the second contact point in three spatial directions, in particular wherein exactly one spatial direction is coupled such that the first and second contact points have a defined distance (99) in this spatial direction.

12. Device (10) according to any of the preceding claims, wherein the element to be brought into engagement with the eye of the user is a contact lens; wherein the holder (30) is a holder for the contact lens and is adapted to apply the contact lens to the eye of the user in the predefined engagement position.

13. System (1) for electrostimulation of the eye, comprising a device (10) according to any of the preceding claims and a stimulation electrode arrangement (20) for electrostimulation of the eye, wherein the element to be brought into engagement with an eye of the user is a stimulation electrode (21) and wherein the holder (30) is an electrode holder for the stimulation electrode (21).

14. Non-surgical method (100), in particular a method for applying a stimulation electrode of a device for electrostimulation of the eye to the eye of the user, wherein the method comprises the following steps:
a) providing (S101) a device (10), in particular a device for electrostimulation of the eye, wherein the device (10) comprises a spectacle-like frame (11) with a nose part (12), an arrangement connected to the nose part (12) for holding the frame on the head of the user, and at least one holder (30) arranged at the frame (11) for an element to be brought into engagement with an eye of the user, in particular for a wire-shaped stimulation electrode (21), wherein the holder (30) is adapted to be moved back and forth between a non-engagement position and a predefined engagement position;
b) placing (S102) the device on the head of the user, wherein the holder is in the non-engagement position; and
c) moving (S103) the holder (30) from the non-engagement position to the predefined engagement position after placing the device on the head of the user, wherein in the predefined engagement position, a further movement of the holder toward the eye of the user is mechanically limited, in particular by a stop, to bring the element (21) to be brought into engagement with the eye of the user into engagement with the eye of the user .

## Revendications

1. Dispositif (10), en particulier dispositif pour l'électrostimulation de l'œil, dans lequel le dispositif (10) présente une monture (11) en forme de lunettes comportant une partie pour le nez (12), un agencement relié à la partie pour le nez (12) et permettant de maintenir la monture sur la tête de l'utilisateur, ainsi qu'au moins un moyen de maintien (30) disposé sur la monture (11) pour un élément à amener en prise avec un œil de l'utilisateur, en particulier pour une électrode de stimulation (21) filiforme, **caractérisé en ce que** le moyen de maintien (30) est conçu pour être déplacé en va-et-vient entre une position de non-mise en prise et une position de mise en prise prédéfinie, dans lequel le moyen de maintien (30) présente une branche verticale nasale (82) et une branche verticale temporale (83), qui s'étendent à partir du moyen de maintien, en particulier à partir d'une traverse horizontale (81) du moyen de maintien, en direction verticale vers le bas, dans lequel la branche verticale nasale (82) et/ou la branche verticale temporale (83) sont réglables en longueur (92, 93).

2. Dispositif (10), en particulier dispositif pour l'électrostimulation de l'œil, dans lequel le dispositif (10) présente une monture (11) en forme de lunettes comportant une partie pour le nez (12), un agencement relié à la partie pour le nez (12) et permettant de maintenir la monture sur la tête de l'utilisateur, ainsi qu'au moins un moyen de maintien (30) disposé sur la monture (11) pour un élément à amener en prise avec un œil de l'utilisateur, en particulier pour une électrode de stimulation (21) filiforme, **caractérisé en ce que** le moyen de maintien (30) est conçu pour être déplacé en va-et-vient entre une position de non-mise en prise et une position de mise en prise prédéfinie, dans lequel le moyen de maintien (30) présente une branche horizontale nasale (84) et une branche horizontale temporale (85), dans lequel la branche horizontale nasale (84) présente un premier logement pour l'élément (20, 21) à amener en prise avec l'œil de l'utilisateur, dans lequel la branche horizontale temporale (85) présente un second logement pour l'élément (20, 21) à amener en prise avec l'œil de l'utilisateur, et dans lequel la branche horizontale nasale (84) et/ou la branche horizontale temporale (85) sont réglables en longueur (95, 96).

3. Dispositif (10) selon la revendication 1 ou 2, dans lequel le dispositif est un dispositif pour l'électrostimulation de l'œil et dans lequel le moyen de maintien (30) est un moyen de maintien d'électrodes pour un agencement d'électrodes de stimulation (20), en particulier dans lequel l'agencement d'électrodes de stimulation (20) présente un premier support d'électrodes (22) et un second support d'électrodes (23), dans lequel l'électrode de stimulation (21) est disposée entre le premier support d'électrodes (22) et le second support d'électrodes (23), dans lequel le premier et/ou le second support d'électrodes sont conçus pour être fixés magnétiquement au moyen de maintien d'électrodes (30).

4. Dispositif (10) selon l'une des revendications précédentes, dans lequel le dispositif présente deux moyens de maintien séparés, un moyen de maintien gauche (30') pour un élément à amener en prise avec un œil gauche de l'utilisateur, en particulier pour une électrode de stimulation (21) pour l'œil gauche, et un moyen de maintien droit (30) pour un élément à amener en prise avec un œil droit de l'utilisateur, en particulier pour une électrode de stimulation (21) pour l'œil droit ; en particulier dans lequel le moyen de maintien gauche (30') et le moyen de maintien droit (30) sont conçus pour être déplacés en va-et-vient séparément l'un de l'autre entre une position de non-mise en prise respective et une position de mise en prise respective.

5. Dispositif (10) selon l'une des revendications précédentes, dans lequel le moyen de maintien (30) est conçu pour être déplacé en va-et-vient entre une position de non-mise en prise prédéfinie réglable et une position de mise en prise prédéfinie réglable ; et/ou
dans lequel, dans la position de mise en prise prédéfinie, un déplacement du moyen de maintien en direction de l'œil de l'utilisateur est limité mécaniquement, en particulier dans lequel la limitation est réglable ; et/ou
dans lequel le moyen de maintien (30) repose, dans la position de mise en prise prédéfinie, contre une butée d'extrémité (58) fournie sur la monture (11).

6. Dispositif (10) selon l'une des revendications précédentes, dans lequel le moyen de maintien (30) présente une poignée d'actionnement (51) pour le déplacement du moyen de maintien entre la position de non-mise en prise et la position de mise en prise, dans lequel
(a) la poignée d'actionnement (51) fait saillie dans la direction horizontale (56) au-delà de la monture (11) ; en particulier dans lequel un élément de manipulation (55) permettant de régler le moyen de maintien (30) dans la direction horizontale (56) par rapport à la monture (11) est disposé dans la poignée d'actionnement (51) ; et/ou
(b) le dispositif est conçu de sorte que la poignée d'actionnement est disposée en dehors d'un champ de vision de l'utilisateur, en particulier en dehors d'un champ de vision central de l'utilisateur ; en particulier dans lequel le dispositif est conçu de sorte que la poignée d'actionnement (51) est disposée en dehors d'un champ de vision central de l'utilisateur dans la position de mise en prise.

7. Dispositif (10) selon l'une des revendications précédentes, le moyen de maintien (30) est conçu pour être déplacé en va-et-vient, d'une seule main et sans outil, entre la position de non-mise en prise et la position de mise en prise prédéfinie.

8. Dispositif (10) selon la revendication 2 ou selon la revendication 2 en combinaison avec l'une des revendications précédentes, dans lequel le premier logement de la branche horizontale nasale (84) et le second logement de la branche horizontale temporale (85) présentent un écartement horizontal fixe (99) l'un par rapport à l'autre.

9. Dispositif (10) selon la revendication 1 et 2, ou selon la revendication 1 et 2 en combinaison avec l'une des revendications précédentes, dans lequel la branche verticale nasale (82) et la branche verticale temporale (83) s'étendent vers le bas dans la direction verticale à partir de la traverse horizontale (81) du moyen de maintien (30) ; dans lequel la branche horizontale nasale (84) est disposée à une extrémité inférieure de la branche verticale nasale (82) ; et dans lequel la branche horizontale temporale (85) est disposée à une extrémité inférieure de la branche verticale temporale (83) ; en particulier dans lequel la branche verticale nasale (82) et la branche verticale temporale (83) sont disposées orthogonalement par rapport à la traverse (81) du moyen de maintien (30) ; dans lequel la branche horizontale nasale (84) et la branche horizontale temporale (85) sont disposées orthogonalement par rapport à la traverse (81) du moyen de maintien ; dans lequel la branche horizontale nasale (84) est disposée orthogonalement par rapport à la branche verticale nasale (82) ; et dans lequel la branche horizontale temporale (85) est disposée orthogonalement par rapport à la branche verticale temporale (83).

10. Dispositif (10) selon l'une des revendications précédentes, dans lequel la branche verticale nasale (82) et/ou la branche verticale temporale (83) et/ou la branche horizontale nasale (84) et/ou la branche horizontale temporale (85) sont réglables en longueur à l'aide d'une tige filetée.

11. Dispositif (10) selon l'une des revendications précédentes, dans lequel le moyen de maintien (30) est conçu pour amener en prise avec l'œil de l'utilisateur l'élément à amener en prise avec l'œil de l'utilisateur en un premier point d'appui et en un second point d'appui dans la position de mise en prise, dans lequel le moyen de maintien (30) est conçu pour ajuster le premier point d'appui dans trois directions spatiales et pour ajuster le second point d'appui dans trois directions spatiales, en particulier dans lequel exactement une direction spatiale est couplée de telle sorte que le premier et le second point d'appui présentent un espacement défini (99) dans ladite direction spatiale.

12. Dispositif (10) selon l'une des revendications précédentes, dans lequel l'élément à amener en prise avec l'œil de l'utilisateur est une lentille de contact ; dans lequel le moyen de maintien (30) est un moyen de maintien pour la lentille de contact et est conçu pour appliquer la lentille de contact sur l'œil de l'utilisateur dans la position de mise en prise prédéfinie.

13. Système (1) pour l'électrostimulation de l'œil comportant un dispositif (10) selon l'une des revendications précédentes et un agencement d'électrodes de stimulation (20) pour l'électrostimulation de l'œil, dans lequel l'élément à amener en prise avec un œil de l'utilisateur est une électrode de stimulation (21) et dans lequel le moyen de maintien (30) est un moyen de maintien d'électrodes pour l'électrode de stimulation (21).

14. Procédé non chirurgical (100), en particulier procédé permettant de mettre en place sur l'œil de l'utilisateur une électrode de stimulation d'un dispositif pour l'électrostimulation de l'œil, dans lequel le procédé présente les étapes suivantes :
a) fourniture (S101) d'un dispositif (10), en particulier pour l'électrostimulation de l'œil, dans lequel le dispositif (10) présente une monture (11) en forme de lunettes comportant une partie pour le nez (12), un agencement relié à la partie pour le nez (12) et permettant de maintenir la monture sur la tête de l'utilisateur, ainsi qu'au moins un moyen de maintien (30) disposé sur la monture (11) pour un élément à amener en prise avec un œil de l'utilisateur, en particulier pour une électrode de stimulation (21) filiforme, dans lequel le moyen de maintien (30) est conçu pour être déplacé en va-et-vient entre une position de non-mise en prise et une position de mise en prise prédéfinie ;
b) mise en place (S102) du dispositif sur la tête de l'utilisateur, dans lequel le moyen de maintien se trouve alors dans la position de non-mise en prise ; et
c) déplacement (S103) du moyen de maintien (30) de la position de non-mise en prise vers la position de mise en prise prédéfinie après la mise en place du dispositif sur la tête de l'utilisateur, dans lequel dans la position de mise en prise prédéfinie un déplacement supplémentaire du moyen de maintien en direction de l'œil de l'utilisateur est limité mécaniquement, en particulier par une butée, afin d'amener l'élément (21) à amener en prise avec l'œil de l'utilisateur en prise avec l'œil de l'utilisateur.
